# EUROPEAN PATENT SPECIFICATION

(11) **EP 4 146 817 B1**
(45) Date of publication and mention of the grant of the patent: **05.08.2026**
(21) Application number: 21800932.2
(22) Date of filing: 07.05.2021
(51) Int. Cl.: C12Q 1/6825, C12Q 1/6886

(54) **LIQUID BIOPSY PLATFORM IN PLASMA AND SALIVA**
FLÜSSIGE BIOPSIEPLATTFORM IN PLASMA UND SPEICHEL
PLATE-FORME DE BIOPSIE LIQUIDE DANS LE PLASMA ET LA SALIVE

(30) Priority: 07.05.2020 US 202063021172 P
(43) Date of publication of application: 15.03.2023
(73) Proprietor: The Regents of the University of California, Oakland, CA 94607 (US); Liao, Wei, Northridge, CA 91326 (US)
(72) Inventor: WONG, David, T., Beverly Hills, CA 90211 (US); WEI, Fang, Northridge, CA 91326 (US); TU, Michael, Cupertino, CA 95014 (US); CHENG, Jordan, Oakland, CA 94607 (US); CHIA, David, Oakland, CA 94607 (US); YE, Qianlin, Oakland, CA 90211 (US); LI, Feng, Oakland, CA 90211 (US); STROM, Charles, San Clemente, CA 92673 (US); LIAO, Wei, Northridge, CA 91326 (US); TANG, Jason, Oakland, CA 94607 (US)
(74) Representative: Duncombe, Jessica
(86) International application number: PCT/US2021/031359
(87) International publication number: WO 2021/226501

(56) References cited:
- WO-A1-2015/187855
- WO-A1-2018/059576
- US-A- 5 891 630
- US-A1- 2010 173 285
- US-A1- 2011 214 206
- US-A1- 2012 282 610
- US-A1- 2014 194 296
- US-B1- 6 391 558
- OBERHAUS ET AL.: "Immobilization Techniques for Aptamers on Gold Electrodes for the Electrochemical Detection of Proteins: A Review", BIOSENSORS, vol. 10, no. 5, 28 April 2020 (2020-04-28), pages 45, XP055891612

## Description

### CROSS REFERENCE TO RELATED APPLICATIONS

This application claims priority to U.S. Provisional Application No. 63/021,172, filed May 7, 2020.

### STATEMENT REGARDING FEDERALLY SPONSORED RESEARCH OR

### DEVELOPMENT

This invention was made with government support under Grant Numbers DE017790, DE017170, CA206126, CA233370, awarded by the National Institutes of Health. The government has certain rights in the invention.

### BACKGROUND OF THE INVENTION

Next generation sequencing (NGS) has unlocked a wealth of new biologic information among which are the characteristic mutations associated with a number of solid tumors including lung, colon, breast and prostate cancer (Almodovar et al., 2018, J Thorac Oncol, 13:112-123; Schwaederle et al., 2016, Clin Cancer Res, 22:5497-5505; Rolfo et al., 2018, J Thorac Oncol, 13:1248-1268). Heretofore, tissue samples obtained by biopsy or at the time of surgery were the only available specimen source. As such, the amount of tissue available was often limiting. More recently, technology has allowed us to detect and measure cell-free DNA (cfDNA) in the blood (Wei et al., 2018, J Mol Diagn, 20:738-742) and saliva (Wei et al., 2014, Am J Respir Crit Care Med, 190:1117-1126; Pu et al., 2016, Thoracic Cancer, 7:428-436; Elazezy et al., 2018, Comput Struct Biotechnol J, 2018, 16:370-378) providing ready access to a limitless supply of specimens. This sampling method is known as "liquid biopsy".

While liquid biopsy is still in its infancy as a clinical tool, it has already demonstrated value in detecting Epidermal growth factor receptor *(EGFR)* mutations in patients with non-small cell lung cancer (NSCLC) at the time of presentation and relapse prompting Roche Molecular Systems to commercially release the Cobas *EGFR* Mutation Test in 2017. In addition, commercial laboratories now offer single or panel mutation tests using cfDNA obtained by liquid biopsy for patients with NSCLC.

To date, the widespread use of liquid biopsy has been negatively impacted by challenges in the sensitivity and specificity of the assay and by its significant cost often exceeding $1000 for a single mutation. Further, CMS-Medicare and other third-party payors have balked at covering such testing with the exception of the Cobas assay which is FDA approved.

The complexity of current liquid biopsy testing introduces a number of both pre-analytic and analytic variables which result from specimen collection and handling to the multiplex PCR testing required. In addition, the complexity of the analysis for Next Generation Sequencing (NGS) based tests requires a great deal of time so that assay turnaround time (TAT) is typically 10-14 days.

WO2018059576 describes a probe for human EGFR genetic mutation detection, a kit and a detection method thereof. WO2015187855 describes a system and method for the detection of saliva biomarkers of lung cancer. US6391558 describes the electrochemical detection of nucleic acid sequences.

Thus, there is a need in the art for high-throughput liquid biopsy systems that are non-invasive, always available, include minimal or no sample preparation, and provide immediate information on mutation status. The present invention satisfies this need.

### SUMMARY OF THE INVENTION

In one embodiment, the invention relates to a system for detecting a nucleic acid molecule of interest in a sample, comprising: a) a multi-well plate comprising an array of sensors, wherein each well comprises an electrode chip including a working electrode, a counter electrode, and a reference electrode; wherein the working electrode of at least one unit is coated with a conducting polymer; b) at least one probe set comprising a paired capture and detector probe, wherein the capture probe comprises a polyA region and is embedded or functionalized in the conducting polymer and further wherein the detector probe is biotin labeled at the terminal 3'nucleotide; c) a multi-well plate washer; and d) a multi-channel electrochemical reader which controls an electrical field applied onto the array sensors and reports the amperometric current simultaneously.

In one embodiment, at least one of the capture and detector probes hybridize to a nucleic acid molecule comprising a marker of lung cancer.

In one embodiment, the marker of lung cancer is a variant of Epidermal growth factor receptor (EGFR). In one embodiment, the marker of lung cancer is Exon19 Deletion, T790M, L858R, c.2235_2249 del18, c.2236_2250 del18, c.2240_2257 del18, c.2239_2248TTAAGAGAAG>C, c.2239_2247delTTAAGAGAA or c.2239_2248 del11.

In one embodiment, the marker of lung cancer is Exon19 Deletion, T790M, or L858R.

In one embodiment, the paired capture and detection probes are: a) paired probes for detection of Exon19 Deletion, wherein the capture probe comprises a nucleotide sequence of SEQ ID NO:5 or SEQ ID NO: 13, and wherein the detector probe comprises SEQ ID NO:6; b) paired probes for detection of L858R, wherein the capture probe comprises a nucleotide sequence of SEQ ID NO:1 or SEQ ID NO:14, and wherein the detector probe comprises SEQ ID NO:2; or c) paired probes for detection of T790M, wherein the capture probe comprises a nucleotide sequence of SEQ ID NO:9 or SEQ ID NO:15, and wherein the detector probe comprises SEQ ID NO:10.

In one embodiment, the invention relates to a method of detecting lung cancer in a subject comprising: providing at least one sample obtained from the subject; mixing a first portion of the at least one sample with a solution comprising a labeled detector probe; adding the mixture to a single well of a multi-well plate for use in a system for detecting a nucleic acid molecule of interest in a sample, comprising: a) a multi-well plate comprising an array of sensors, wherein each well comprises an electrode chip including a working electrode, a counter electrode, and a reference electrode; wherein the working electrode of at least one unit is coated with a conducting polymer; b) at least one probe set comprising a paired capture and detector probe, wherein the capture probe comprises a polyA region and is embedded or functionalized in the conducting polymer and further wherein the detector probe is biotin labeled at the terminal 3'nucleotide; c) a multi-well plate washer; and d) a multi-channel electrochemical reader which controls an electrical field applied onto the array sensors and reports the amperometric current simultaneously, wherein each well of the multi-well plate comprises an electrode chip comprising a working electrode, a counter electrode, and a reference electrode; wherein the working electrode is coated with a conducting polymer embedded with a capture probe, wherein the capture probe comprises a polyA region and; applying a cyclic square-wave electric field to the electrode chip; adding a first round of streptavidin bound horseradish peroxidase (HRP) to the well, adding a biotin labeled anti-HRP antibody to the well, adding a second round of streptavidin bound HRP to the well, and measuring the current in the electrode chip, wherein a change in current is correlated to the presence of a marker associated with lung cancer in the sample.

In one embodiment, the method further comprises at least one washing step, wherein the multi-well plate is washed using an automated plate washer.

In one embodiment, at least one reagent is maintained at 4°C prior to use.

In one embodiment, at least one of the capture and detector probes hybridize to a nucleic acid molecule comprising a marker of lung cancer. In one embodiment, the nucleic acid molecule is a circulating tumor DNA (ctDNA) molecule.

In one embodiment, the marker of lung cancer is a variant of EGFR. In one embodiment, the marker of lung cancer is Exon19 Deletion, T790M, L858R, c.2235_2249 del18, c.2236_2250 del18, c.2240_2257 del18, c.2239_2248TTAAGAGAAG>C,
c.2239_2247delTTAAGAGAA or c.2239_2248 del11. In one embodiment, the marker of lung cancer is Exon19 Deletion, T790M, or L858R.

In one embodiment, the paired capture and detection probes are: a) paired probes for detection of Exon19 Deletion, wherein the capture probe comprises a nucleotide sequence of SEQ ID NO:5 or SEQ ID NO: 13, and wherein the detector probe comprises SEQ ID NO:6; b) paired probes for detection of L858R, wherein the capture probe comprises a nucleotide sequence of SEQ ID NO:1 or SEQ ID NO: 14, and wherein the detector probe comprises SEQ ID NO:2; or c) paired probes for detection of T790M, wherein the capture probe comprises a nucleotide sequence of SEQ ID NO:9 or SEQ ID NO:15, and wherein the detector probe comprises SEQ ID NO:10.

In one embodiment, at least one sample is a saliva sample, a blood sample, a plasma sample or a serum sample.

In one embodiment, a saliva sample from a subject is added to a first well of the multi-well plate and a plasma sample from the same subject is added to a second well of the multi-well plate. In one embodiment, a subject is diagnosed as having or being at risk lung cancer when a maker of lung cancer is detected in both the saliva sample from the subject and the plasma sample from the same subject.

### BRIEF DESCRIPTION OF THE DRAWINGS

The following detailed description of exemplary embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.

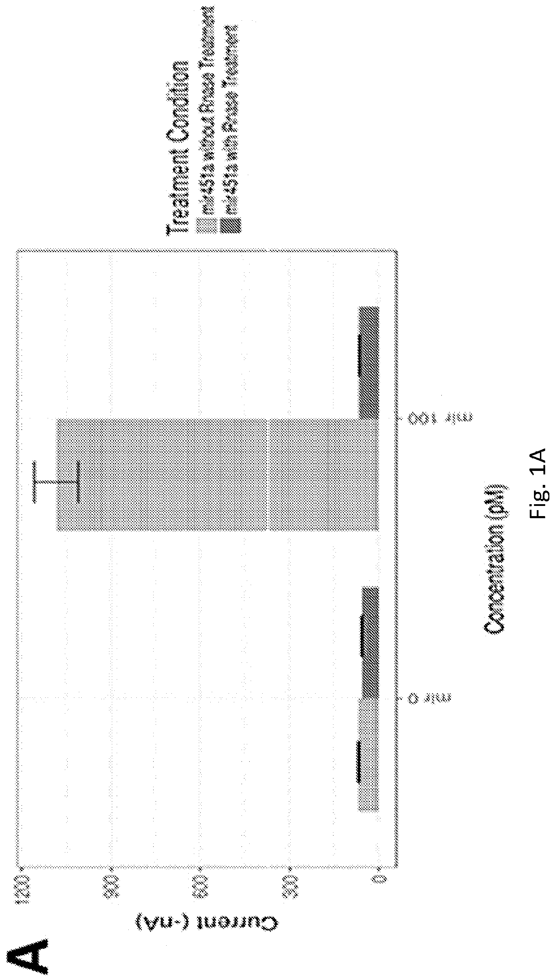

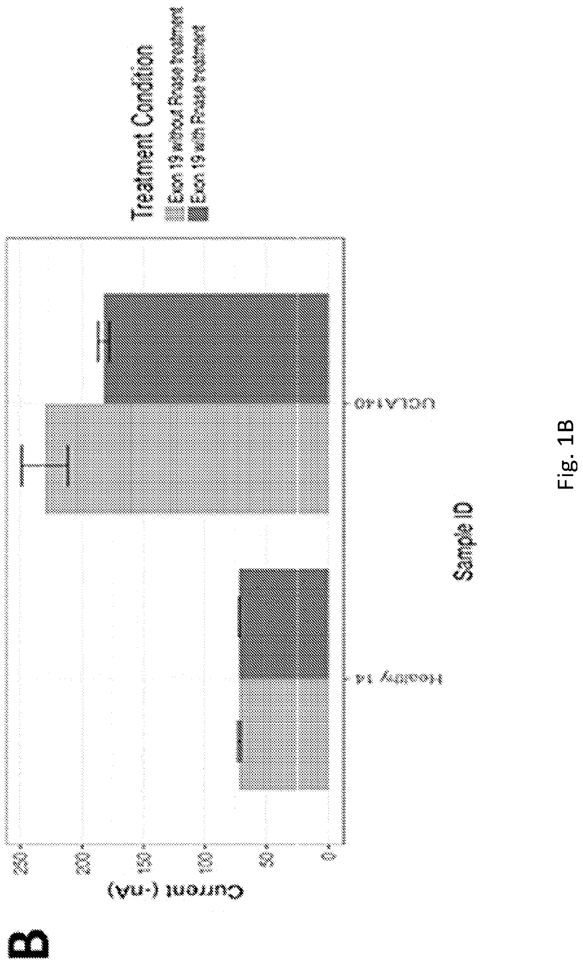

Figure 1A and Figure 1B depict exemplary eLB assay experiment with Rnase treatment to evaluate whether the target detected by eLB is DNA or RNA. Figure 1A: RNAse treatment experiment applied to an RNA based assay. Figure 1B: RNAse treatment applied to a healthy control and a subject (UCLA140) bearing Exon19 Deletion.

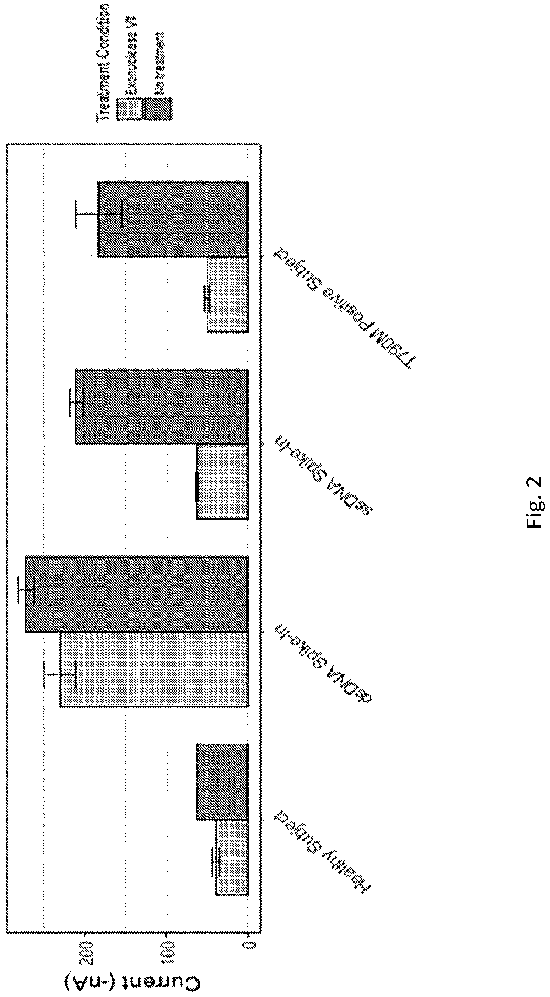

Figure 2 depicts an exemplary eLB assay experiment with exonuclease VII treatment to evaluate whether the target detected by eLB is DNA or RNA. Exonuclease VII treatment was applied to a healthy sample; a healthy plasma sample with a synthetic double stranded T790M oligonucleotide spike-in; a healthy plasma with a synthetic single stranded T790M spike-in; or a *p. T790M* bearing subject.

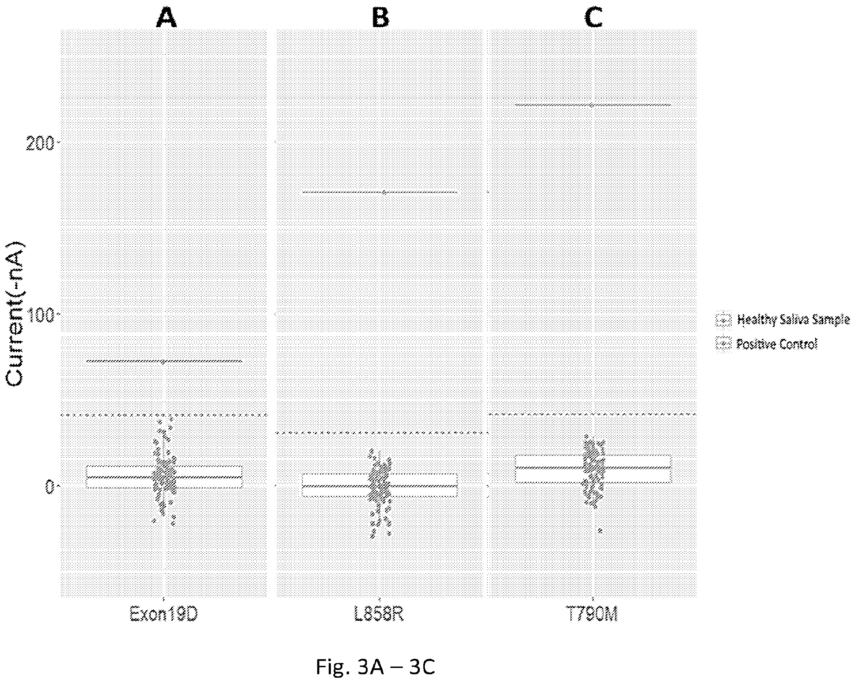

Figure 3A through Figure 3C depict exemplary experiments demonstrating a reference range study for 100 healthy saliva samples tested using eLB assay. 2 standard deviation line indicated by the dotted line. Figure 3A: eLB assay for Exon19 deletion mutation; Figure 3B: eLB assay results for p.L858R mutation; Figure 3C: eLB assay for p.T790M mutation.

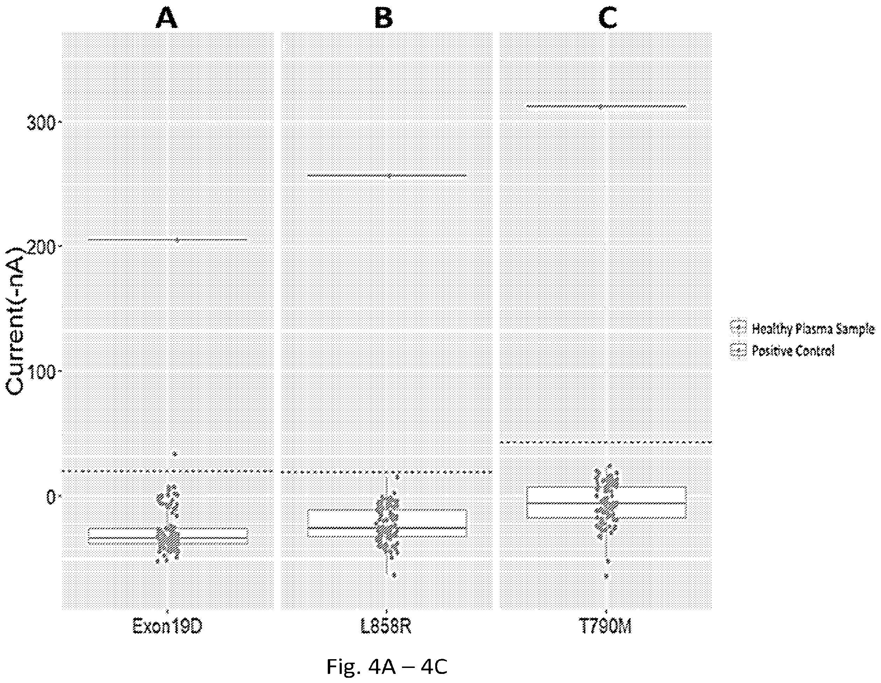

Figure 4A through Figure 4C depict exemplary experiments demonstrating a reference range study for 100 healthy plasma samples tested using eLB assay. 2 standard deviation line indicated by red-dotted line. Figure 4A: eLB assay for Exon19 deletion mutation; Figure 4B: eLB assay results for p.L858R mutation; Figure 4C: eLB assay for p.T790M mutation.

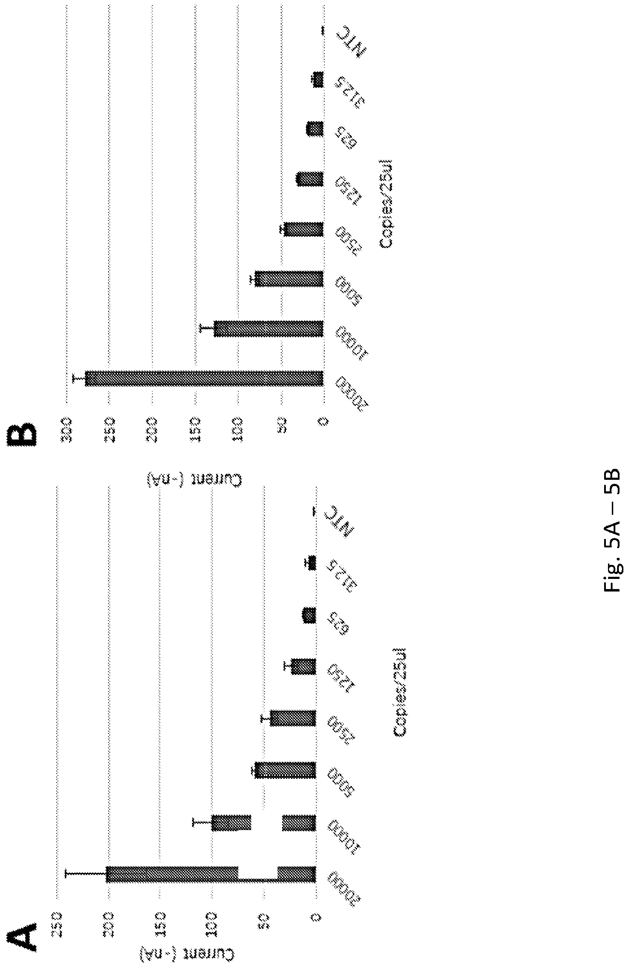

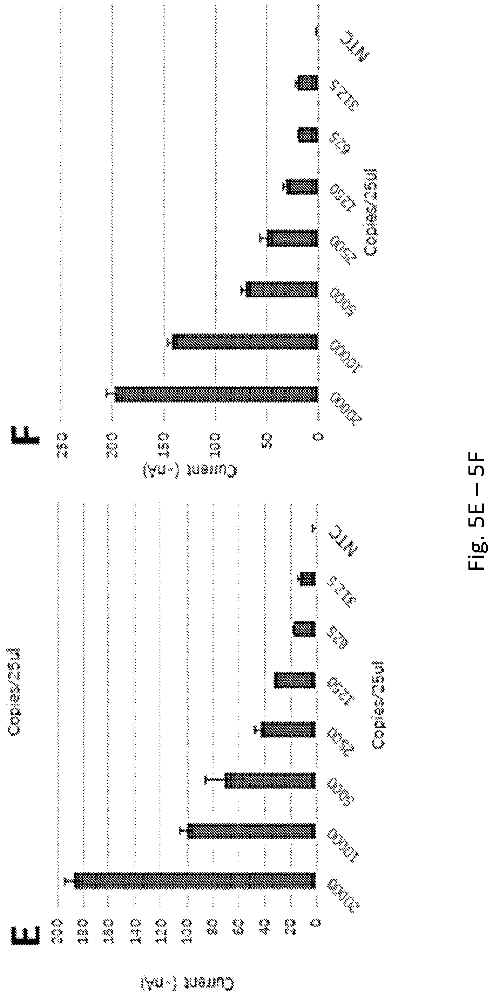

Figure 5A through Figure 5H depict exemplary experiments demonstrating an eLB assay results of serially diluted genomic DNA for the top four EGFR exon19 variants. Figure 5A through Figure 5D depict plots of measurement results for variants c.2235_2249 del18, c2236_2250 del18, c2240_2257 del18, and c.2239_2248 del11, respectively. Figure 5E through Figure 5H depict the linear range of c.2235_2249 del18, c2236_2250 del18, c2240_2257 del18, and c.2239 _2248 del11, respectively.

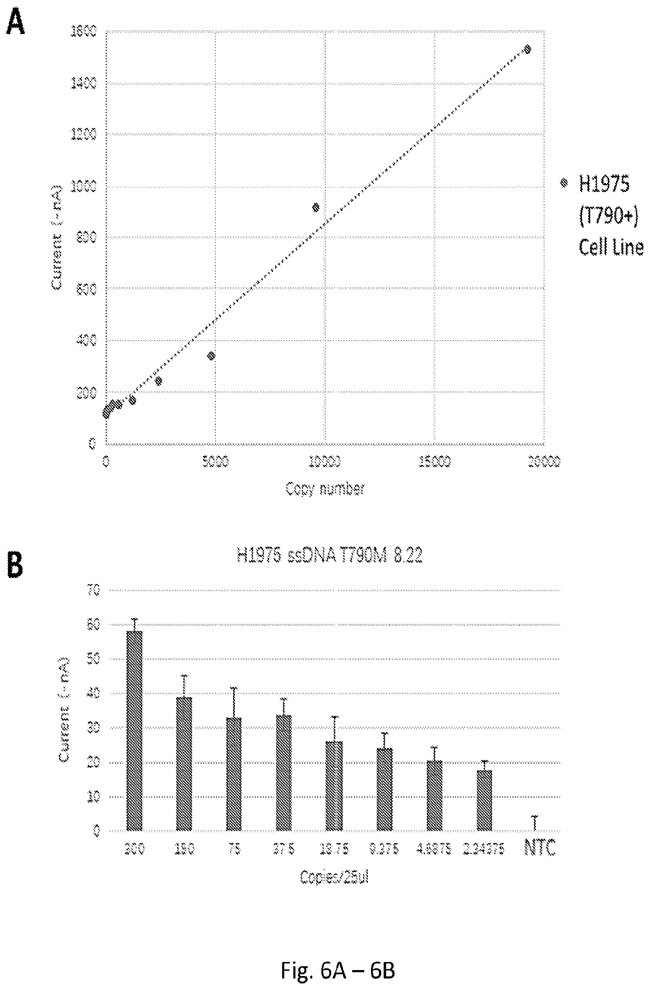

Figure 6A and Figure 6B depict exemplary eLB assay results of serially diluted genomic DNA for p.T790M mutations. Figure 6A depicts a scatter-plot of diluted genomic DNA and EFIRM measurements Figure 6B depicts a bar-plot of the lower ranges (300 to 2 copies) of the serially diluted genomic DNA.

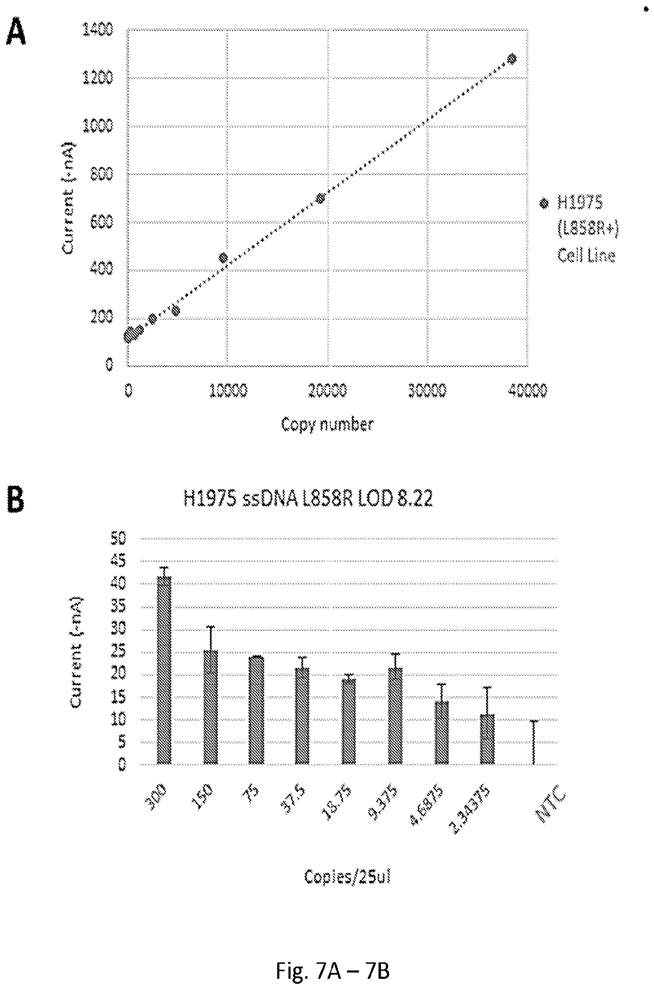

Figure 7A and Figure 7B depict exemplary eLB assay results of serially diluted genomic DNA for p.L858R mutations. Figure 7A depicts a scatter-plot of diluted genomic DNA and EFIRM measurements. Figure 7B depicts a bar-plot of the lower ranges (300 to 2 copies) of the serially diluted genomic DNA.

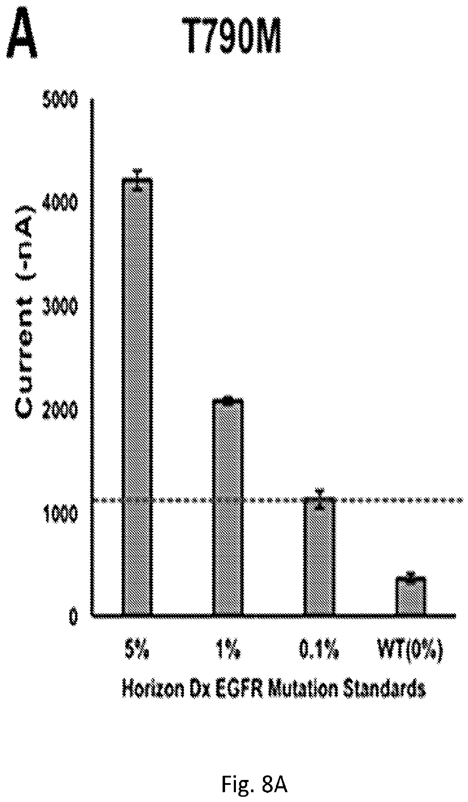

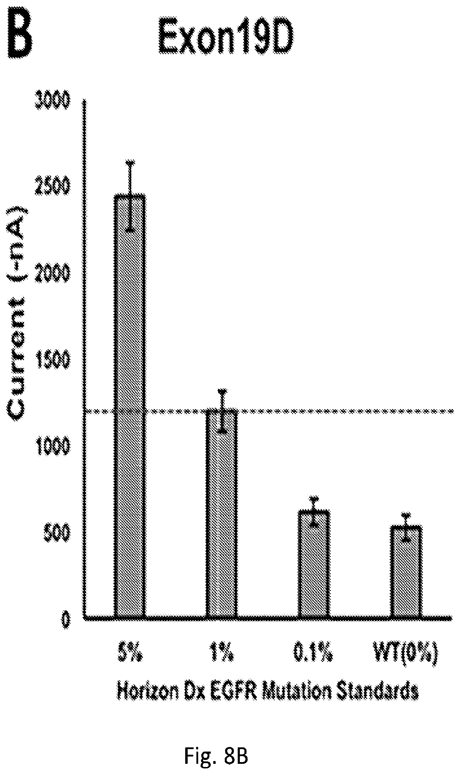

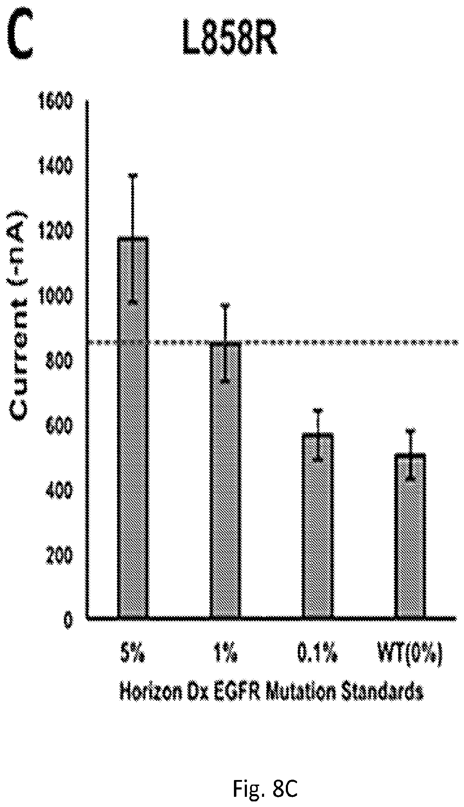

Figure 8A through Figure 8C depict exemplary eLB assay results of standards from Horizon Diagnostics reference standards. Figure 8A: eLB for p.T790M; Figure 8B: eLB for Exon19 Deletion; Figure 8C: eLB for p.L858R.

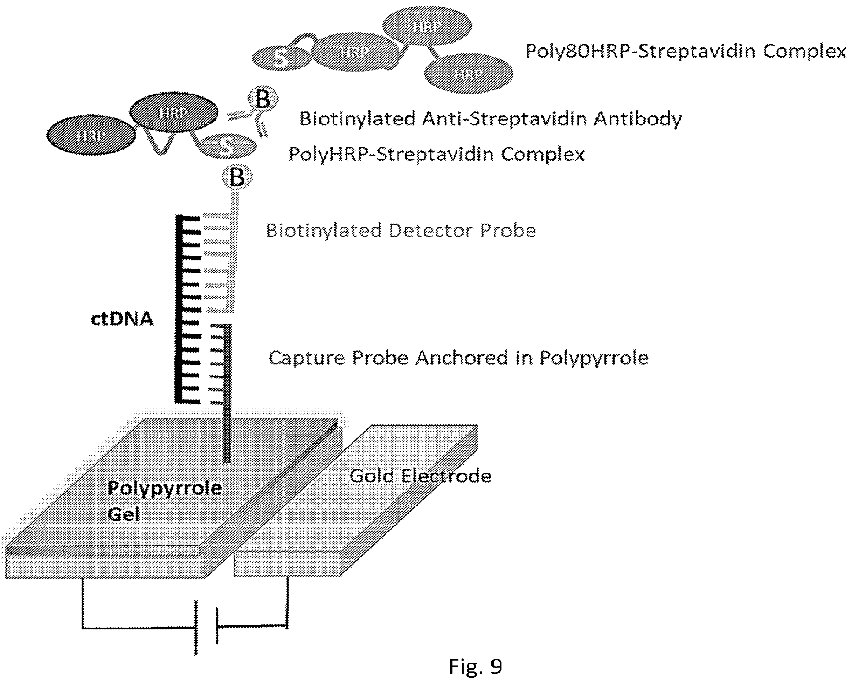

Figure 9 depicts a schematic diagram of the EFIRM assay system.

### DETAILED DESCRIPTION

It is to be understood that the figures and descriptions of the present invention have been simplified to illustrate elements that are relevant for a clear understanding of the present invention, while eliminating, for the purpose of clarity, many other elements found in typical biomarker detection systems and methods. Those of ordinary skill in the art may recognize that other elements and/or steps are desirable and/or required in implementing the present invention. However, because such elements and steps are well known in the art, and because they do not facilitate a better understanding of the present invention, a discussion of such elements and steps is not provided herein. The disclosure herein is directed to all such variations and modifications to such elements and methods known to those skilled in the art.

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, exemplary methods and materials are described.

As used herein, each of the following terms has the meaning associated with it in this section.

The articles "a" and "an" are used herein to refer to one or to more than one *(i.e.,* to at least one) of the grammatical object of the article. By way of example, "an element" means one element or more than one element.

"About" as used herein when referring to a measurable value such as an amount, a temporal duration, and the like, is meant to encompass variations of ±20%, ±10%, ±5%, ±1%, and ±0.1% from the specified value, as such variations are appropriate.

The term "abnormal" when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics which are normal or expected for one cell or tissue type, might be abnormal for a different cell or tissue type.

As used herein the terms "alteration," "defect," "variation," or "mutation," refers to a mutation in a gene in a cell that affects the function, activity, expression (transcription or translation) or conformation of the polypeptide that it encodes. Mutations encompassed by the present invention can be any mutation of a gene in a cell that results in the enhancement or disruption of the function, activity, expression or conformation of the encoded polypeptide, including the complete absence of expression of the encoded protein and can include, for example, missense and nonsense mutations, insertions, deletions, frameshifts and premature terminations. Without being so limited, mutations encompassed by the present invention may alter splicing the mRNA (splice site mutation) or cause a shift in the reading frame (frameshift).

The term "amplification" refers to the operation by which the number of copies of a target nucleotide sequence present in a sample is multiplied.

The term "antibody," as used herein, refers to an immunoglobulin molecule which specifically binds with an antigen. Antibodies can be intact immunoglobulins derived from natural sources or from recombinant sources and can be immunoreactive portions of intact immunoglobulins. Antibodies are typically tetramers of immunoglobulin molecules. The antibodies in the present invention may exist in a variety of forms including, for example, polyclonal antibodies, monoclonal antibodies, Fv, Fab and F(ab)₂, as well as single chain antibodies and humanized antibodies (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426).

An "antibody heavy chain," as used herein, refers to the larger of the two types of polypeptide chains present in all antibody molecules in their naturally occurring conformations.

An "antibody light chain," as used herein, refers to the smaller of the two types of polypeptide chains present in all antibody molecules in their naturally occurring conformations. κ and λ light chains refer to the two major antibody light chain isotypes.

By the term "synthetic antibody" as used herein, is meant an antibody which is generated using recombinant DNA technology, such as, for example, an antibody expressed by a bacteriophage as described herein. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using synthetic DNA or amino acid sequence technology which is available and well known in the art.

By the term "specifically binds," as used herein with respect to an antibody, is meant an antibody which recognizes a specific antigen, but does not substantially recognize or bind other molecules in a sample. For example, an antibody that specifically binds to an antigen from one species may also bind to that antigen from one or more species. But, such cross-species reactivity does not itself alter the classification of an antibody as specific. In another example, an antibody that specifically binds to an antigen may also bind to different allelic forms of the antigen. However, such cross reactivity does not itself alter the classification of an antibody as specific. In some instances, the terms "specific binding" or "specifically binding," can be used in reference to the interaction of an antibody, a protein, or a peptide with a second chemical species, to mean that the interaction is dependent upon the presence of a particular structure (e.g., an antigenic determinant or epitope) on the chemical species; for example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody.

As used herein, the term "marker" or "biomarker" is meant to include a parameter which is useful according to this invention for determining the presence and/or severity of lung cancer.

The level of a marker or biomarker "significantly" differs from the level of the marker or biomarker in a reference sample if the level of the marker in a sample from the patient differs from the level in a sample from the reference subject by an amount greater than the standard error of the assay employed to assess the marker, for example, by at least 5%, 10%, 25%, 50%, 75%, or 100%.

The term "control or reference standard" describes a material comprising none, or a normal, low, or high level of one of more of the marker (or biomarker) expression products of one or more the markers (or biomarkers) of the invention, such that the control or reference standard may serve as a comparator against which a sample can be compared.

By the phrase "determining the level of marker (or biomarker) expression" is meant an assessment of the degree of expression of a marker in a sample at the nucleic acid or protein level, using technology available to the skilled artisan to detect a sufficient portion of any marker expression product.

"Differentially increased expression" or "up regulation" refers to biomarker product levels which are at least 10% or more, for example, 20%, 30%, 40%, or 50%, 60%, 70%, 80%, 90% higher or more, and/or 1.1 fold, 1.2 fold, 1.4 fold, 1.6 fold, 1.8 fold, 2.0 fold higher or more, and any and all whole or partial increments therebetween than a control.

"Differentially decreased expression" or "down regulation" refers to biomarker product levels which are at least 10% or more, for example, 20%, 30%, 40%, or 50%, 60%, 70%, 80%, 90% lower or less, and/or 2.0 fold, 1.8 fold, 1.6 fold, 1.4 fold, 1.2 fold, 1.1 fold or less lower, and any and all whole or partial increments therebetween than a control.

A "disease" is a state of health of an animal wherein the animal cannot maintain homeostasis, and wherein if the disease is not ameliorated then the animal's health continues to deteriorate.

As used herein, an "instructional material" includes a publication, a recording, a diagram, or any other medium of expression which can be used to communicate the usefulness of a component of the invention in a kit for detecting biomarkers disclosed herein. The instructional material of the kit of the invention can, for example, be affixed to a container which contains the component of the invention or be shipped together with a container which contains the component. Alternatively, the instructional material can be shipped separately from the container with the intention that the instructional material and the component be used cooperatively by the recipient.

The term "label" when used herein refers to a detectable compound or composition that is conjugated directly or indirectly to a probe to generate a "labeled" probe. The label may be detectable by itself (e.g. radioisotope labels or fluorescent labels) or, in the case of an enzymatic label, may catalyze chemical alteration of a substrate compound or composition that is detectable (e.g., avidin-biotin). In some instances, primers can be labeled to detect a PCR product.

The "level" of one or more biomarkers means the absolute or relative amount or concentration of the biomarker in the sample.

The term "marker (or biomarker) expression" as used herein, encompasses the transcription, translation, post-translation modification, and phenotypic manifestation of a gene, including all aspects of the transformation of information encoded in a gene into RNA or protein. By way of non-limiting example, marker expression includes transcription into messenger RNA (mRNA) and translation into protein, as well as transcription into types of RNA such as transfer RNA (tRNA) and ribosomal RNA (rRNA) that are not translated into protein.

"Measuring" or "measurement," or alternatively "detecting" or "detection," means assessing the presence, absence, quantity or amount (which can be an effective amount) of either a given substance within a clinical or subject-derived sample, including the derivation of qualitative or quantitative concentration levels of such substances, or otherwise evaluating the values or categorization of a subject's clinical parameters.

The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is a human.

As used herein, the term "providing a prognosis" refers to providing a prediction of the probable course and outcome of lung cancer, including prediction of severity, duration, chances of recovery, etc. The methods are used to devise a suitable therapeutic plan, e.g., by indicating whether or not the condition is still at an early stage or if the condition has advanced to a stage where aggressive therapy would be ineffective.

A "reference level" of a biomarker means a level of the biomarker that is indicative of a particular disease state, phenotype, or lack thereof, as well as combinations of disease states, phenotypes, or lack thereof. A "positive" reference level of a biomarker means a level that is indicative of a particular disease state or phenotype. A "negative" reference level of a biomarker means a level that is indicative of a lack of a particular disease state or phenotype.

"Sample" or "biological sample" as used herein means a biological material isolated from an individual. The biological sample may contain any biological material suitable for detecting the desired biomarkers, and may comprise cellular and/or non-cellular material obtained from the individual.

"Standard control value" as used herein refers to a predetermined amount of a particular protein or nucleic acid that is detectable in a sample, such as a saliva sample, either in whole saliva or in saliva supernatant. The standard control value is suitable for the use of a method of the present invention, in order for comparing the amount of a protein or nucleic acid of interest that is present in a saliva sample. An established sample serving as a standard control provides an average amount of the protein or nucleic acid of interest in the saliva that is typical for an average, healthy person of reasonably matched background, e.g., gender, age, ethnicity, and medical history. A standard control value may vary depending on the protein or nucleic acid of interest and the nature of the sample (e.g., whole saliva or supernatant).

Throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, 6 and any whole and partial increments therebetween. This applies regardless of the breadth of the range.

### Description

The present invention relates to a rapid and accurate polymer-based electrochemical platform array for single or multi-biomarker detection from at least one biological sample, such as a saliva sample or blood sample, that are indicative of a disease or disorder, for example, lung cancer. While the present invention is described generally for the testing of a saliva sample or blood sample, it should be appreciated that any biological fluid sample may be used, or even other tissue types, provided such alternative sample types carry the targeted markers to be analyzed. Non-limiting examples of such markers include all saliva and serum-based markers of the disease or disorder. In some embodiments, the marker is a marker of lung cancer, including but not limited to, a nucleic acid molecule carrying a gene mutation, such as one or more mutations in epidermal growth factor receptor (EGFR), or any other markers associated with or indicative of lung cancer. For example, markers that are indicative of lung cancer, or detectible mutations in genes, include but are not limited to Exon19 Deletion, T790M, L858R, c.2235_2249 del18, c.2236_2250 del18, c.2240_2257 del18, c.2239_2248TTAAGAGAAG>C, c.2239_2247delTTAAGAGAA and c.2239_2248 del11 variants of EGFR. It should be appreciated that any number of biomarkers can be integrated to the assay platform, including, without limitation, 1, 2, 4, 8, 16, 32 or 64 biomarkers per array.

The noninvasive detection of lung cancer in a subject via the present invention enables clinicians to identify the presence of lung cancer in a fast, economical and non-invasive manner.

As contemplated herein, the present invention includes a multiplexing electrochemical sensor for detecting biomarkers. The device utilizes a small sample volume with high accuracy. In addition, multiple markers can be measured simultaneously on the device with single sample loading. The device may significantly reduce the cost to the health care system, by decreasing the burden of patients returning to clinics and laboratories.

In one embodiment, the electrochemical sensor is an array of electrode chips (EZ Life Bio, USA). In one embodiment, each unit of the array has a working electrode, a counter electrode, and a reference electrode. The three electrodes may be constructed of bare gold or other conductive material before the reaction, such that the specimens may be immobilized on the working electrode. Electrochemical current can be measured between the working electrode and counter electrode under the potential between the working electrode and the reference electrode. The potential profile can be a constant value, a linear sweep, or a cyclic square wave, for example. An array of plastic wells may be used to separate each three-electrode set, which helps avoid the cross contamination between different sensors. **In** one embodiment, a three-electrode set is in each well of a 96 well gold electrode plate. A conducting polymer may also be deposited on the working electrodes as a supporting film, and in some embodiments, as a surface to functionalize the working electrode. As contemplated herein, any conductive polymer may be used, such as polypyrroles, polanilines, polyacetylenes, polyphenylenevinylenes, polythiophenes and the like.

**In** one embodiment, a cyclic square wave electric field is generated across the electrode within the sample well. **In** certain embodiments, the square wave electric field is generated to aid in polymerization of one or more capture probes to the polymer of the sensor. **In** certain embodiments, the square wave electric field is generated to aid in the hybridization of the capture probes with the marker and/or detector probe. The positive potential in the csw E-field helps the molecules accumulate onto the working electrode, while the negative potential removes the weak nonspecific binding, to generate enhanced specificity. Further, the flapping between positive and negative potential across the cyclic square wave also provides superior mixing during incubation, without disruption of the desired specific binding, which accelerates the binding process and results in a faster test or assay time. **In** one embodiment, a square wave cycle may consist of a longer low voltage period and a shorter high voltage period, to enhance binding partner hybridization within the sample. While there is no limitation to the actual time periods selected, examples include 0.15 to 60 second low voltage periods and 0.1 to 60 second high voltage periods. **In** one embodiment, each square-wave cycle consists of 1 s at low voltage and 1 s at high voltage. For hybridization, the low voltage may be around -200 mV and the high voltage may be around +500 mV. **In** some embodiments, the total number of square wave cycles may be between 2-50. **In** one embodiment, 5 cyclic square-waves are applied for each surface reaction. With the csw E-field, both the polymerization and hybridization are finished on the same chip within minutes. **In** some embodiments, the total detection time from sample loading is less than 30 minutes. **In** other embodiments, the total detection time from sample loading is less than 20 minutes. **In** other embodiments, the total detection time from sample loading is less than 10 minutes. **In** other embodiments, the total detection time from sample loading is less than 5 minutes. In other embodiments, the total detection time from sample loading is less than 2 minutes. In other embodiments, the total detection time from sample loading is less than 1 minute.

A multi-channel electrochemical reader (EZ Life Bio) controls the electrical field applied onto the array sensors and reports the amperometric current simultaneously. In practice, solutions can be loaded onto the entire area of the three-electrode region including the working, counter, and reference electrodes, which are confined and separated by the array of plastic wells. After each step, the electrochemical sensors can be rinsed with ultrapure water or other washing solution and then dried, such as under pure N₂. In some embodiments, the sensors are single use, disposable sensors. In other embodiment, the sensors are reusable.

In one embodiment, the present invention is based on the affinity between a capture probe and a detector probe, as shown in Figure 9. As contemplated herein, the assay platform may be organized as any type of affinity binding assay or immunoassay, as would be understood by those skilled in the art. In another embodiment, the present invention includes a single platform for multiple lung cancer biomarker measurements, instead of a single marker. Currently, there is no such technology or device available for this purpose. In another embodiment, the present invention creates tremendous efficiencies in that it is simple, rapid and robust. For example, only small sample volumes are needed (e.g., 10 µl) and less than 10 minutes run time are needed. Multiple marker levels may be provided by the device. By providing statistical analysis the user may have an estimate of their risk, and by utilizing available networking systems, the results can be quickly transmitted for review by a clinician for further assessment.

For example, paired probes (capture and detector) specific for a mutation can be designed, such as for a deletion mutation, or for a point mutation. In one embodiment, a paired probe set of the invention for detection of an *Exon 19del* EGFR variant comprises a capture probe comprising SEQ ID NO: 13 and a detector probe comprising SEQ ID NO:6. In one embodiment, a paired probe set of the invention for detection of a *L858R* EGFR variant comprises a capture probe comprising SEQ ID NO: 14 and a detector probe comprising SEQ ID NO:2. In one embodiment, a paired probe set of the invention for detection of a *T790M* EGFR variant comprises a capture probe comprising SEQ ID NO:15 and a detector probe comprising SEQ ID NO: 10.

In some embodiments, at least one of the capture and detector probes comprises a polyA tail. In various embodiments, the polyA tail comprises at least 40, 45, 50, 55, 60, 65, 70, 75, 80, or more than 80 adenine nucleotides at the 5' or 3' end of the probe. In some embodiments, the polyA tail comprises a sequence comprising at least 65 nucleotides, wherein at least 80% of the nucleotides are adenine, and wherein the polyA sequence is interrupted by non-adenine nucleotides. In one embodiment, a paired probe set of the invention for detection of a *L858R* EGFR variant comprises a capture probe comprising SEQ ID NO: 1 and a detector probe comprising SEQ ID NO:2. In one embodiment, a paired probe set of the invention for detection of an *Exon 19del* EGFR variant comprises a capture probe comprising SEQ ID NO:5 and a detector probe comprising SEQ ID NO:6. In one embodiment, a paired probe set of the invention for detection of an T790M EGFR variant comprises a capture probe comprising SEQ ID NO:9 and a detector probe comprising SEQ ID NO:10.

The detector probes can be labeled, such as with fluorescein isothiocyanate, or any other label known in the art. In one embodiment, the detector probes contain a biotinylated nucleotide to allow streptavidin binding. The capture probe is first copolymerized onto the bare gold electrode by applying a cyclic square wave electric field. For example, for each cycle during copolymerization, the electric field can be set to +350 mV for 1 s and +950 mV for 1 s. In total, polymerization may proceed for 5 cycles of 10 s, or however long is deemed necessary.

After polymerization, the sensor chip can be rinsed and dried for subsequent sample measurement. Samples, such as a cell-culture medium, a blood sample or a saliva sample, can be mixed with the detector probes and transferred onto the electrodes. Hybridization is then carried out at low and high voltage cycles, such as -200 mV for 1 s and +500 mV for 1 s. The total hybridization time can be 5 cycles for 10 s, for example. Next, the label is detected based on the label type. For example, an anti-fluorescein antibody conjugated to horseradish peroxidase in casein-phosphate-buffered saline can be used, and the 3,3',5,5'-tetramethylbenzidine substrate for horseradish peroxidase can be loaded, and the amperometric signal measured.

Capture probes embedded in the conductive polymer or otherwise used to functionalize the working electrode surface, and detector probes mixed with the sample may be constructed according to any protocol known in the art for the generation of probes.

In one embodiment, capture probes are immobilized in a conductive polymer gel in the bottom of the 96 well gold electrode plate.

The capture probe or detector probe of the sensor may be any one of a nucleic acid, protein, small molecule, and the like, which specifically binds one or more of the markers of interest. For example, in a particular embodiment, the capture probe and detector probe are oligonucleotides or polynucleotides comprising a region that is substantially complementary to one or more nucleic acid markers of the invention. **In** one embodiment, the capture probe and detector probe comprise a region that is substantially complementary to each other. That is, in one embodiment, the capture probe comprises a region that is substantially complementary to a region of the detector probe. Methods for designing and formulating oligonucleotide probes are well-known in the art.

**In** one embodiment, the marker comprises a variable region, where the variable region may comprise a mutation, such as a deletion, substitution, point mutation, and the like, associated with the disease. **In** one embodiment, the capture probe is designed to hybridize to a conserved region of the nucleic acid marker (i.e. present in wild-type and mutant isoforms). **In** one embodiment, the detector probe is designed to hybridize to the nucleic acid sequence of the variable region having a disease-associated mutation of the marker, thereby detecting the mutation in the sample. **In** another embodiment, the detector probe is designed to hybridize to the nucleic acid sequence of the variable region having a wild-type or non-mutated sequence, thereby detecting the presence of the wildtype marker in the sample. **In** one embodiment, the capture probe is designed to hybridize to the wildtype or mutated variable region, while the detector probe is designed to hybridize to the conserved region.

For example, in one embodiment, the capture probe comprises a nucleotide sequence that is substantially complementary to a conserved region of a nucleic acid encoding EGFR. **In** one embodiment, the detector probe comprises a nucleotide sequence that is substantially complementary to a variable region of a nucleic acid encoding EGFR, where the variable region encodes the wild-type amino acid sequence of EGFR. **In** one embodiment, the detector probe comprises a nucleotide sequence that is substantially complementary to a variable region of a nucleic acid encoding EGFR, where the variable region encodes the disease-associated mutant sequence of EGFR. For example, in certain embodiments, the detector probe comprises a nucleotide sequence that is substantially complementary to a variable region of a nucleic acid encoding EGFR, where the variable region encodes the disease-associated deletion mutant or point mutation of EGFR.

In one embodiment, when the present invention is used for detecting a nucleic acid encoding the L858R mutant of EGFR, the capture probe comprises a nucleic acid molecule comprising the nucleotide sequence of SEQ ID NO: 1 or SEQ ID NO: 14. In one embodiment, when the present invention is used for detecting a nucleic acid encoding the L858R mutant of EGFR, the detector probe comprises a nucleic acid molecule comprising the nucleotide sequence of SEQ ID NO: 2.

In one embodiment, when the present invention is used for detecting a nucleic acid encoding the Exon19 del mutant of EGFR, the capture probe comprises a nucleic acid molecule comprising the nucleotide sequence of SEQ ID NO: 5 or SEQ ID NO: 13. In one embodiment, when the present invention is used for detecting a nucleic acid encoding the Exon19 del mutant of EGFR, the detector probe comprises a nucleic acid molecule comprising the nucleotide sequence of SEQ ID NO: 6.

In one embodiment, when the present invention is used for detecting a nucleic acid encoding the T790M mutant of EGFR, the capture probe comprises a nucleic acid molecule comprising the nucleotide sequence of SEQ ID NO: 9 or SEQ ID NO: 15. In one embodiment, when the present invention is used for detecting a nucleic acid encoding the T790M mutant of EGFR, the detector probe comprises a nucleic acid molecule comprising the nucleotide sequence of SEQ ID NO: 10.

In one embodiment, the detector probe comprises a detectable label which induces a change in current of the sensor, thereby indicating the hybridization of the detector probe, and associated marker, with the capture probe. In certain embodiments, the detectable label itself may be sufficient to alter the current of the sensor. In certain embodiments, the detectable label induces the change in current when it comes into contact with an exogenous reactant. For example, the detectable label may react with the reactant to produce a local change sensed by the electrodes of the sensor to produce an amperometric signal. Therefore, in certain embodiments, the reactant is added to the sensor prior, during, or after the application of the sample to the sensor.

In certain embodiments, the detectable label is directly conjugated to the detector probe. In another embodiment, the detectable label is bound to the detector probe via an intermediate tag or label of the probe. In some embodiments, the detectable label is a modified nucleotide containing biotin incorporated into the detector probe during synthesis. For example, in one embodiment, the detector probe comprises a tag, label, or epitope, which can be used to bind to an antibody or other binding compound harboring the detectable label described above.

Examples of detectable labels and reactants to produce a local change in an electrochemical sensor are well known in the art. In one embodiment, the detectable label comprises HRP and the reactant is TMB, which react to generate an amperometric signal. In another embodiment, the detectable label comprises urease, while the reactant comprises urea.

In one embodiment, the signal is amplified using multiple rounds of HRP. In on embodiment, 1) a biotin labeled detector molecule is contacted with a first round of HRP in the form of streptavidin bound HRP, 2) the complexed HRP molecule is contacted with a biotin labeled Anti-HRP antibody, and 3) a second round of streptavidin bound HRP is added to amplify the signal. In one exemplary embodiment, the detector probes are mixed with casein-phosphate-buffered saline at a 1:100 dilution and transferred onto the electrodes. Hybridization is performed at 300 mV for 1 second and 500 mV for 1 second for 150 cycles at room temperature. Subsequently, streptavidin Poly-HRP is mixed with casein-phosphate-buffered saline at a 1:1000 ratio and incubated on the electrodes for 30 minutes at room temperature. After the addition of HRP, Anti-HRP antibody with casein-phosphate-buffered saline is added, followed by a 30 minute incubation at room temperature and a wash-off with PBS-T buffer. Subsequently, Streptavidin Poly-HRP80 Conjugate mixed with casein-phosphate-buffered saline is added and incubated for 30 minutes to increase the amount of available HRP molecules. This method, results in increased signal amplification, allowing for increased sensitivity and specificity of the eLB system. In some embodiments, one or more washing steps are performed. In some embodiments, the plate is washed in an automated 96 well plate washer, in which the existing liquid is aspirated from each well of the microtiter plate, and then a wash butter dispensed into each well. In one embodiment, the wash buffer is then aspirated and this is repeated for at least one additional cycle.

Due to the enhanced sensitivity of the present invention, very small volumes may be used to perform the desired assays. For example, the biological sample size from the subject may be between 5-100 microliters. In one embodiment, the sample size need only be about 40 microliters. There is no limitation to the actual or final sample size to be tested.

The present invention also relates to a method of detecting one or more markers associated with or indicative of lung cancer in a subject. In one embodiment, the method may be performed as a hybridization assay and includes the steps of obtaining a sample from the subject, adding a detector probe labeled with a detectable moiety directed against a targeted marker of lung cancer to the sample, applying the sample to an electrode chip coated with a conducting polymer previously embedded or functionalized with the capture probe, and measuring the current in the electrode chip. The detectable moiety may be measured, or the magnitude of the current in the sample may be measured, to determine the presence or absence of the marker in the sample. In certain embodiments, hybridization of the marker to the electrode of the sensor results in an increase in current or negative current. For example, in one embodiment, hybridization results in a current in the range of about -10nA to about -1000nA.

In one embodiment, the liquid and enzymatic reagents used in the methods of the invention are refrigerated at 4°C when not in use. In one embodiment, the sample and the reagents used in the methods of the invention are maintained on ice prior to use. In one embodiment, the sample is processed within 30 minutes of collection. The present invention provides a method for diagnosing lung cancer in a subject. Accordingly, the present invention features methods for identifying subjects who are at risk of developing lung cancer, including those subjects who are asymptomatic or only exhibit non-specific indicators of lung cancer by detection of the biomarkers disclosed herein. These biomarkers are also useful for monitoring subjects undergoing treatments and therapies for lung cancer, and for selecting or modifying therapies and treatments that would be efficacious in subjects having lung cancer, wherein selection and use of such treatments and therapies slow the progression of lung cancer, or prevent their onset.

In certain embodiments, the biomarkers detected by way of the system and method of the invention include, but are not limited to nucleic acids, or detectible mutations in genes including those associated with EGFR, KRAS, BRAF, CCNI, FGF19, FRS2, GREB1 and LZTS1. In certain embodiments, the nucleic acid biomarkers comprise one or more disease-associated mutations, including, insertions, deletions, substitutions, translocations, point mutations, single nucleotide variants (SNVs), and the like. The present invention may be used to detect any disease-associated biomarker or disease-associated mutation known in the art or discovered in the future. Exemplary mutations present in various forms of cancer may be found, for example, in the Catalogue of Somatic Mutations in Cancer (COSMIC) (Wellcome Trust Sanger Institute).

In certain embodiments, the biomarkers detected by way of the system and method of the present invention comprise mutant EGFR and nucleic acid molecules encoding mutant EGFR. For example, various EGFR mutants are associated with lung cancer, including, but not limited to, Exon19 Deletion, T790M, L858R, c.2235_2249 del18, c.2236_2250 del18, c.2240_2257 del18, c.2239_2248TTAAGAGAAG>C, c.2239_2247delTTAAGAGAA and c.2239 _2248 del11 variants of EGFR.

The present invention may be used to detect any such mutation, as the capture probe and/or detector probe may be specifically designed to hybridize to a nucleic acid molecule encoding the mutant protein of interest.

The invention provides improved diagnosis, therapeutic monitoring, detection of recurrence, and prognosis of lung cancer. The risk of developing lung cancer can be assessed by measuring one or more of the biomarkers described herein, and comparing the measured values to reference or index values. Such a comparison can be undertaken with mathematical algorithms or formula in order to combine information from results of multiple individual biomarkers and other parameters into a single measurement or index. Subjects identified as having an increased risk of lung cancer can optionally be selected to receive treatment regimens, such as administration of prophylactic or therapeutic compounds or treatments to prevent, treat or delay the onset of lung cancer.

Identifying a subject before they develop lung cancer enables the selection and initiation of various therapeutic interventions or treatment regimens in order to delay, reduce or prevent the development or severity of the cancer. In certain instances, monitoring the levels of at least one biomarker also allows for the course of treatment of lung cancer to be monitored. For example, a sample can be provided from a subject undergoing treatment regimens or therapeutic interventions, e.g., drug treatments, radiation, chemotherapy, etc. for lung cancer. Samples can be obtained from the subject at various time points before, during, or after treatment.

The biomarkers of the present invention can thus be used to generate a biomarker profile or signature of subjects: (i) who do not have and are not expected to develop lung cancer and/or (ii) who have or expected to develop lung cancer. The biomarker profile of a subject can be compared to a predetermined or reference biomarker profile to diagnose or identify subjects at risk for developing lung cancer, to monitor the progression of disease, as well as the rate of progression of disease, and to monitor the effectiveness of lung cancer treatments. Data concerning the biomarkers of the present invention can also be combined or correlated with other data or test results for lung cancer, including but not limited to imaging data, medical history, smoking status and any relevant family history.

The present invention also provides methods for identifying agents for treating lung cancer that are appropriate or otherwise customized for a specific subject. **In** this regard, a test sample from a subject, exposed to a therapeutic agent, drug, or other treatment regimen, can be taken and the level of one or more biomarkers can be determined. The level of one or more biomarkers can be compared to a sample derived from the subject before and after treatment, or can be compared to samples derived from one or more subjects who have shown improvements in risk factors as a result of such treatment or exposure.

**In** one embodiment, the invention is a method of diagnosing lung cancer. **In** one embodiment, the method includes determining the stage or severity of lung cancer. **In** some embodiments, these methods may utilize at least one biological sample (such as urine, saliva, blood, serum, plasma, amniotic fluid, or tears), for the detection of one or more markers of the invention in the sample. Frequently the sample is a "clinical sample" which is a sample derived from a patient. **In** one embodiment, the biological sample is a blood sample. **In** certain embodiments, the biological sample is a serum sample or a plasma sample, derived from a blood sample of the subject.

**In** some embodiments, the sample comprises at least one ultra-small single stranded DNA molecule. **In** one embodiment, the sample comprises at least on circulating tumor DNA (ctDNA) molecule. **In** one embodiment, the ctDNA comprises a biomarker associated with cancer.

**In** one embodiment, the method comprises detecting one or more markers in at least one biological sample of the subject. **In** various embodiments, the level of one or more of markers of the invention in the biological sample of the subject is compared with the level of a corresponding biomarker in a comparator. Non-limiting examples of comparators include, but are not limited to, a negative control, a positive control, an expected normal background value of the subject, a historical normal background value of the subject, an expected normal background value of a population that the subject is a member of, or a historical normal background value of a population that the subject is a member of.

**In** one embodiment, the method comprises detecting one or more markers simultaneously in two or more different biological samples of the subject. **In** one embodiment, the method comprises detecting one or more markers simultaneously in a saliva sample of the subject and a blood, plasma or serum sample of the subject.

**In** one embodiment, the method comprises detecting one or more markers sequentially in two or more different biological samples of the subject. **In** one embodiment, the method comprises detecting one or more markers in a saliva sample of the subject prior to or subsequently to detecting one or more markers in a blood, plasma or serum sample of the subject.

**In** various embodiments, the level of one or more of markers of the invention in each biological sample of the subject is compared with the level of a corresponding biomarker in an appropriate comparator. **In** one embodiment, a subject is diagnosed as having or being at risk of a disease or disorder when the level of the biomarker in each of two or more biological samples is increased or decreased relative to the appropriate comparator. For example, in one embodiment, the subject is diagnosed as having or being at risk of lung cancer when 1) the level of at least one DNA molecule containing a mutation associated with lung cancer is increased in a saliva sample of the subject relative to an expected normal background level of the biomarker in the saliva of a healthy subject or the healthy population, and 2) the level of at least one DNA molecule containing a mutation associated with lung cancer is increased in a plasma sample of the subject relative to an expected normal background level of the biomarker in the serum of a healthy subject or the healthy population.

**In** another embodiment, the invention is a method of monitoring the progression of lung cancer in a subject by assessing the level of one or more of the markers of the invention in at least one biological sample of the subject.

**In** various embodiments, the subject is a human subject, and may be of any race, sex and age.

Information obtained from the methods of the invention described herein can be used alone, or in combination with other information (e.g., disease status, disease history, vital signs, blood chemistry, etc.) from the subject or from the biological sample obtained from the subject.

**In** other various embodiments of the methods of the invention, the level of one or more markers of the invention is determined to be increased when the level of one or more of the markers of the invention is increased by at least 10%, by at least 20%, by at least 30%, by at least 40%, by at least 50%, by at least 60%, by at least 70%, by at least 80%, by at least 90%, or by at least 100%, when compared to with a comparator control.

**In** the methods of the invention, at least one biological sample from a subject is assessed for the level of one or more of the markers of the invention in the biological sample obtained from the patient. The level of one or more of the markers of the invention in the biological sample can be determined by assessing the amount of polypeptide of one or more of the biomarkers of the invention in the biological sample, the amount of mRNA of one or more of the biomarkers of the invention in the biological sample, the amount of DNA of one or biomarkers of the invention in the biological sample, the amount of enzymatic activity of one or more of the biomarkers of the invention in the biological sample, or a combination thereof.

The present invention further includes an assay kit containing the electrochemical sensor array and instructions for the set-up, performance, monitoring, and interpretation of the assays of the present invention. Optionally, the kit may include reagents for the detection of at least one of the biomarkers. The kit may also optionally include the sensor reader.

### EXPERIMENTAL EXAMPLES

The invention is further described in detail by reference to the following experimental examples. These examples are provided for purposes of illustration only, and are not intended to be limiting unless so specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the present invention and practice the claimed methods. The following working examples therefore, specifically point out exemplary embodiments of the present invention, and are not to be construed as limiting in any way the remainder of the disclosure.

### Example 1: Noninvasive EGFR Gene Mutation Detection in Patients with Lung Cancer

Currently there are 2 popular liquid biopsy platforms, next generation sequencing based (NGS) and droplet digital PCR (ddPCR) based (Zhang et al., 2017, J Hematol Oncol, 10:167; Olmedillas-Lopez et al., 2017, Mol Diagn Ther, 21:493-510). Both of these platforms require 10 - 20 mL of blood to perform the analysis. This report describes the technical validation of 4 separate assays using a novel, plate based, electrochemical signal amplification method for the purposes of liquid biopsy, referred to herein as eLB. Early reported data has demonstrated that eLB is capable of detecting circulating *EGFR* variant DNA in patients with early stage lung cancer. This is the only platform capable of this level of discrimination in Stage I and II NSCLC patients. The eLB is preformed using only 20 µL of plasma for each variant. This is more than 100 times less sample required than for either NGS or ddPCR based liquid biopsies.

The eLB is performed on untreated, unpurified plasma or saliva. Both ddPCR and NGS require a DNA isolation step prior to assay. It is likely that a large percentage of the circulating tumor DNA fragments are lost during the DNA isolation procedures or subsequent manipulations. NGS requires several enzymatic treatments and purifications between steps and small fragments of DNA are likely lost during these procedural steps. In addition, the enzymatic steps prior to library formation are not 100% efficient so material is lost at each step due to incomplete reactions.

The usual assumption is that ctDNA fragments are similar in size as the fetal sequences found circulation in the maternal circulation in the noninvasive prenatal screening process. These 150 bp double stranded fragments are the result of nucleosome protection of apoptotic DNA. It is possible, or even likely, that ctDNA does not arise from orderly apoptosis. In fact, most cancer cells lack the programmed cell death that results in apoptotic DNA fragments. Unlike fetal DNA in the maternal circulation, ctDNA is more likely the result of necrosis from outgrowing the blood supply or immune lysis. It is possible that this would result in smaller fragments and potentially single stranded DNA fragments.

In fact, there is evidence that in early stage cancers ctDNA containing actionable mutations is smaller in size than both the wild type DNA and the ctDNA present in advanced cancer (Liu et al., 2019, EBioMedicine, 41:345-56). This report demonstrated that ctDNA from early stage pancreatic cancer patients had a fragment size of significantly less than 100 bp. This study utilized single stranded DNA sequencing strategies so it is possible that the observed ultrashort ctDNA fragments were not only smaller than 100 base pairs, but were also single stranded. Other studies using single stranded sequencing have also demonstrated that ctDNA is smaller in size than that of circulating DNA from other origins (Burnham et al., 2016, Sci Rep, 2016, 6:27859; Underhill et al., 2016, PLOS Genet, 12:e1006162). Preliminary work for our laboratory indicates that the detected mutant ctDNA sequences in NSCLC patients is actually between 35 - 44 bp in length. Other modalities such as ddPCR are not able to detect fragments this small. The unique property of the eLB platform is that its preferred template is ultra-small single stranded DNA allowing eLB to preferentially detect ctDNA while ignoring circulating DNA from other sources.

Another possibility is that the eLB is measuring exosomal DNA. Fernando and colleagues (Fernando et al.,2018, Clin Chim Acta, 483:39-47) report that the majority of exosomal DNA is approximately 76 bp in length. Further work will be necessary to delineate the origin and nature of the fragments analyzed by the eLB.

Another advantage of eLB over other technologies is the ability to use non-standard sample types. In this report both a qualitative and a quantitative eLB assay on saliva was able to be validated It is probable that other samples types such as urine, pleural and cerebral spinal fluid would be amenable to the eLB analysis.

The EFIRM methodology is plate based and the entire assay can be performed easily in 4 hours with very little hands-on time. Therefore, turnaround time is far superior to NGS based methods that often take several days for completion and 10 - 14 days to report and is also more rapid that ddPCR assays.

A final advantage of eLB is the cost. An eLB can be performed for a few hundred dollars and, therefore, could be used for serial monitoring, either for treatment response, early detection of recurrence, or evaluating minimal residual disease. If sensitivities and specificities are sufficient, eLB could also be used for screening smokers or individuals with indeterminate pulmonary nodules on CT scan. The sensitivity for eLB as a screening test currently is limited by the prevalence of the 3 variants among NSCLC patients. Although the frequency is as high as 40% in China (Zhang et al., 2016, Oncotarget, 7:78985-93), the fraction of NSCLC tumors harboring one of these 3 mutations is lower in the United States. The incidence of EGFR mutations is 2.7% with confidence that the true incidence does not exceed 3.6% according to Forbes SA et al. (Curr. Protoc. Hum. Genet., Hoboken, NJ, USA, John Wiley & Sons, Inc., 2008, p. hg1011s57), For adenocarcinoma the overall frequency is 23%. For men it is 19% and for women it is 28%. As for smokers it is 47% overall and 14% for never smokers (Midha et al., 2015, Am J Cancer Res, 2015, 5:2892-911).

In order to increase the sensitivity of detection, additional variants are added to the eLB that increases the detection rate to 50% of NSCLC patients. In summary, this report shows the technical validation of 4 eLB assays: Qualitative and Quantitative assays for plasma and saliva. The platform is stable and suitable for a clinical laboratory. The assay has a minor allele fraction detection of 0.1% for p.T790M and 1% for Exon19del and p.L858R. These specificities are far better than those reported by the only FDA cleared *in vitro* diagnostic for these EGFR variants.

The materials and methods used in the experiments are now described.

### eLB Assay

The eLB assay is an open platform signal amplification technology based on a microtiter plate of 96 gold electrodes and an EFIRM (Electric Field Induced Release and Measurement) electrochemical reader device (EZLife Bio). Three TKI-sensitizing *EGFR* variants, *Exon 19del, p.L858R* and *p. T790M* were validated using the eLB assay. Briefly described, the assay uses variant specific capture probes immobilized in a conductive polymer gel in the bottom of the 96 well gold electrode plate. The sample (either saliva or plasma) is added directly to a hybridization buffer without prior DNA isolation or other manipulation. The sample - hybridization mixture is then added directly into the well and alternating current applied to facilitate specific hybridization at room temperature. The plate is then washed extensively to remove unbound fragments. Subsequently, a biotinylated detector probe in hybridization buffer is added to the wells and subjected to electrically facilitated hybridization. Paired probes (capture and detector) were synthesized (Integrated DNA Technologies) specific for the three TKI-sensitive mutations are listed in Table 1.

The capture and detector probes for the 3 *EGFR* variants are listed below. The * indicates biotinylation of the 3' nucleotide.
L858R Capture Probe (SEQ ID NO:1) 
AAAAAAAAAAGAAATAAACAAATAAAACAATAACAAATAAAAAAAA
ACAAATAAACAATAAAAAAAAACAAGTTTGACCCGCCCA
L858R Detector Probe (for 15 base pair capture probe) (SEQ ID NO:2)
   AAAATCTGTGATCTTGACATGCTGCGGTGTTTTGTGCAG*
Control / calibrator oligo L858R wild type (SEQ ID NO:3) 
CTGGTGAAAACACCGCAGCATGTCAAGATCACAGATTTTGGGCTGGCC
AAACTG
Control / calibrator oligo L858R mutant (SEQ ID NO:4) 
CTGGTGAAAACACCGCAGCATGTCAAGATCACAGATTTTGGGCGGGCC
AAACTG
Exon19 del Capture Probe (SEQ ID NO:5) 
AAAAAAAAAAAAAATAAAAAAAAAAAAAATAAAAAAAAAAAAAATAAAA
AAAAAAAAAATAAAAAAAAAACATGTTGCTTCCTTG
Exon19 del Detector Probe (SEQ ID NO:6)
   ATAGCGACGGGAATTTTAACTTTCTCACCT*
Control / calibrator oligo Exon19 Mutation Del (SEQ ID NO:7) 
AGGTGAGAAAGTTAAAATTCCCGTCGCTATCAAGGAAGCAACATCTCC
GAAAGCCAA
Control / calibrator oligo Exon19 del Wild type (SEQ ID NO:8) 
AGGTGAGAAAGTTAAAATTCCCGTCGCTATCAAGGAATTAAGAGAAG
CAACATCTCCGAAAGCCA
T790M Capture Probe (SEQ ID NO:9) 
AAAAAAAAAAGAAATAAACAAATAAAACAATAACAAATAAAAAAAA
ACAAATAAACAATAAAAAAAAACAAGAGCGGCATGATGA
T790M Detector Probe (SEQ ID NO:10)
   GCTGCACGGTGGAGGTGAGGCAGATGCCCAGC*
Control / calibrator oligo T790M (SEQ ID NO:11) 
GCTGGGCATCTGCCTCACCTCCACCGTGCAGCTCATCATGCAGCTCAT
GCCC
Control / calibrator oligo T790M Wildtype Sequence (SEQ ID NO:12)

GCTGGGCATCTGCCTCACCTCCACCGTGCAGCTCATCACGCAGCTCAT
GCCC

The capture probes (100 nM) were first co-polymerized with pyrrole (W336805, Sigma-Aldrich) onto the bare gold electrodes by applying a cyclic square wave electric field at 350 mV for 1 second and 1100 mV for 1 second. In total, polymerization proceeded for 4 cycles of 2 seconds each.

Synthetic 50 bp oligonucleotides (Integrated DNA Technologies) corresponding to the mutant alleles were added to 20 µl - 40 µl volume of control plasma and saliva (patient samples from individuals who did not harbor the respective EGFR mutations). The synthetic oligonucleotides and plasma or saliva were diluted in Ultrahyb-Oligo Hybridization Buffer (ThermoFisher) at 1:2 ratio. The resulting diluted buffer was added to duplicate wells.

Hybridization was performed at 300 mV for 1 second and 500 mV for 1 second for a total 150 cycles of 2 seconds each followed by a 30-minute incubation period in the eLB instrument at room temperature. The plate was then removed and washed in an automated 96 well plate washer (Biotek, Model 405LSR, Winooski, VT) wherein initially existing liquid is aspirated from each well of the microtiter plate, and then 500 µL of wash butter (2x SSC with 0.5% SDS) is dispensed into each well. The wash buffer is aspirated and this is repeated for a total of 2 cycles.

After washing, detector probes were mixed with casein-phosphate-buffered saline (Invitrogen) at a 1:100 dilution and transferred onto the electrodes. Hybridization was performed at 300 mV for 1 second and 500 mV for 1 second for 150 cycles at room temperature. Subsequently, streptavidin Poly HRP (ThermoFisher) was mixed with casein-phosphate-buffered saline (Invitrogen) at a 1:1000 ratio and incubated on the electrodes for 30 minutes at room temperature.

After the addition of HRP, Anti-HRP antibody (ab195239, Abcam) at a 1/60 µg/mL concentration with casein-phosphate-buffered saline (Invitrogen) was added followed by a 30 minute incubation at room temperature and a wash-off with PBS-T buffer. Subsequently, Streptavidin Poly-HRP80 Conjugate (Fitzgerald Industries) mixed with casein-phosphate-buffered saline (Invitrogen) at a 25:975 ratio was added and incubated for 30 minutes to increase the amount of available HRP molecules. Following this procedure, the amplified signal was measured in nano-amperes by current generation through the gold electrodes. The EFIRM^{®} reader (EZLife Bio), used for electrochemical measurement, is capable of detecting current in the pico-ampere range so that this is well within the technical capability of the instrument.

**Table 1: Capture and Detection Oligonucleotides for eLB EGFR Assay.**

| Variant | Capture Probe |
|---|---|
| *Exon 19del* | 5' - TGTTGCTTCCTTG - 3' (SEQ ID NO:13) |
| *p.L858R* | 5' - GTTTGACCCGCCCA - 3' (SEQ ID NO:14) |
| *p.T790M* | 5' - GAGCGGCATGATGA - 3' (SEQ ID NO:15) |

| | Detector Probe* |
|---|---|
| *Exon 19del* | 5' - ATAGCGACGGGAATTTTAACTTTCTCACCT - 3'* (SEQ ID NO:6) |
| *p.L858R* | 5' - AAAATCTGTGATCTTGACATGCTGCGGTGTTTTGTGCAG - 3'* (SEQ ID NO:2) |
| *p.T790M* | 5' - GCTGCACGGTGGAGGTGAGGCAGATGCCCAGC - 3' * (SEQ ID NO:10 |

| | |
|---|---|
| *Detector probes are biotin labeled at the terminal 3'nucleotide. | |

### RNase Treatment Method

RNase treatment was performed by adding RNase cocktail (0.025U/µl of RNase A and 1U/µl of T1 RNase) in Casein/PBS and incubating for 30 minutes at room temperature. Wash-off was performed using 2x SSC 0.5% SDS wash buffer. This treatment was performed following the sample capture step of the eLB protocol.

### DNase Treatment Method

DNase treatment was performed in order to assess the strandedness of the DNA. First, Proteinase K was added to achieve a concentration of 2 µg/µl in plasma samples. The samples were then incubated for 30 minutes at 50°C. Following this digest, proteinase K was then heated to 65°C for 10 minutes to deactivate the enzyme and then the solution was cooled to 4C for 1 hour (using a cooling rate of 0.1 °C/second) in a thermocycler. Following this initial proteinase K digest to remove interfering proteins, Exonuclease VII was introduced at a concentration of 0.33 unit/µl and incubated at 37°C for 30 minutes. Exonuclease was subsequently deactivated by heat treatment at 95°C for 10 minutes followed by cooling to 4°C for 1 hour (with a ramp rate of 0.1°C/second) to reanneal the double stranded DNA present in the samples. The final solution was then assayed in the EFIRM protocol described above.

### Cell lines

To generate genomic DNA reference standards for the point mutations p.L858R and p. T790M, genomic DNA was isolated using a QuickgDNA miniprep (Zymo Research) from the NCI-H1975 cell line (ATCC). For exon19del testing, genomic DNA from four different cell lines harboring the top four variants of Exon19del mutations (COSM6623/6225/12370/12382) were acquired (Applied Stem Cell).

### Software

The multichannel EFIRM electrochemical reader device hardware is controlled via a USB 2.0 connection and parameters are set by a custom software suite in house by EZLife Bio (Los Angeles, CA). Data from each EFIRM experiment was exported to the comma-separated value file format and analyzed using the R-Language for statistical analysis.

### Biological Samples

Saliva was collected from healthy individual volunteers at meetings of the American Dental Association between 2006 and 2011. There was a mixture of male/females, mostly non-smokers, between 18-80 years of age, and a mixture of ethnicities. All subjects were consented prior to collecting. Each subject would expectorate ~ 5 mL of whole saliva in a 50cc conical tube set on ice. The whole saliva was processed within 1/2 hour of collection. Samples were centrifuged in a refrigerated centrifuge at 2600 g for 15 minutes set to 4°C. The supernatant (cell-free saliva) was then pipetted into two-2 mL cryotubes and the following reagents were added to preserve the RNA and DNA: 1.1 µL Superase-In/1mL supernatant (Ambion, Austin TX). After the additional reagents were added, each tube was inverted to mix. The samples were then frozen on dry ice and later stored in -80°C freezers for storage.

The results of the experiments are now described

### DNA or RNA?

The EFIRM technology is capable of detecting either DNA or RNA molecules. Whether the *EGFR* variants detected in clinical samples from patients with NSCLC were derived for circulating DNA or RNA was investigated. To investigate this, an RNase treatment method was developed as described above. An eLB assay for the microRNA species mir415a was used as a control for completeness of digestion. In the control experiment, synthetic mir415a was added to normal plasma and subjected to eLB analysis both before and after RNase treatment. This result is shown in Figure 1A. The left panel is without the spike in of mi415a and the right panel is with mir415a added. In both panels of Figure 1A the red represents eLB signal without RNase treatment and the blue is following RNase treatment. As can be clearly seen in the right panel of Figure 1A, the RNase treatment completely eliminates the signal from the eLB reaction for the microRNA confirming that the RNase digestion has gone to completion.

The left panel of Figure 1B represents pre and post-treated normal plasma samples analyzed for the Exon19del variant in *EGFR.* The right panel shows plasma from a NSCLC patient from a previously published study with advanced NSCLC disease whose tumor was positive for Exon19del and whose eLB results were also positive (Pu et al., 2016, Thorac Cancer, 7:428-436). There is only a 10% reduction in signal following RNase treatment indicating that the circulating nucleic acid measured in the eLB assay is primarily DNA and not RNA.

### Single Stranded versus Double Stranded DNA

Next, whether the molecule measured by EFIRM is single stranded or double stranded DNA was investigated. For these experiments an enzymatic treatment with the single strand specific nuclease Exonuclease VII was used. Figure 2 contains data from 3 control experiments and an analysis of a clinical sample. Figure 2A shows plasma from a healthy control, demonstrating only background signal in both untreated and Exonuclease VII digested samples. Figure 2B demonstrates that when dsDNA containing the variant is spiked into plasma, that there is no reduction in signal following Exonuclease VII digestion. As expected, when synthetic ssDNA is added, exonuclease VII digestion results in a significant reduction in eLB signal (Figure 2C). Figure 2D represents a plasma sample from an NSCLC patient with advanced whose prior tissue and eLB results demonstrated the p.T790M *EGFR* variant. The exonuclease VII treatment resulted in dramatic reduction in eLB signal similar to the reduction observed in the ssDNA spike in experiment shown in Figure 2C. These data, taken together demonstrate that the eLB is primarily measuring single stranded DNA targets.

### Technical Validation of Qualitative eLB Assay for 3 EGFR Variants

One use for eLB is to screen for malignancies in either asymptomatic individuals, with a smoking history, or in individuals with indeterminate nodules discovered by screening radiographic examination. For this purpose, a "positive" or "negative" result is sufficient. For this purpose, a technical validation of a qualitative eLB was performed for both plasma and saliva.

Exon19del is not a single variant, but a family of closely related variants of slightly varying lengths in Exon 19. Because of the nature of eLB, it was possible to design a capture probe - signal probe combination that detects most, if not all the Exon19del variants. Data presented below that demonstrates that the single capture probe - detector probe pair is capable of detecting the 4 most common Exon19del variants. A further discussion of this issue is found in the quantitative assay description.

### Plasma Qualitative Assay

For validation of a qualitative assay, plasma was purchased from 100 healthy individuals as described elsewhere herein. These samples were run in duplicate on the eLB technology for 3 *EGFR* variants, Exon19del, p.L858R, and p.T790M. The results are shown in Figure 3. Current in nanoamperes (nA) is plotted on the Y axis. The samples are healthy plasma samples with the nA normalized by subtracting the mean nA of the two no template controls (NTC) in each plate. A standard 2 SD reference range is plotted as the dotted line. The solid line is a control oligonucleotide added to each plate to confirm assay performance. The assay performance is appropriate for a qualitative assay. The established reference range varies slightly with each variant. For the plasma qualitative assay, the cut-off values are shown in Table 2 for Exon19del, p.L858R, and p.T790M respectively. The reference range was set to be below 3 SD of the unaffected controls. As can be seen in Figure 3, the internal standard is well above the cutoff values for each variant.

Both inter-assay and intra-assay variability experiments were performed for the qualitative assay using the derived reference range described above. For intra-assay variability a negative and positive sample were run multiple times on a single plate. Each sample was assayed at least 8 times (8 replicates of mutation negative controls and 12 replicates of mutation positive controls were tested for each of the 3 mutations). There were no discrepancies with all negative samples being less than the cut off and all positive samples being above the cutoff.

For inter-assay variability, the mutation negative controls and mutation positive controls (12pM oligonucleotide spiked in biofluid) were run on 3 different plates run by 2 different operators (each plate having 8 replicates of mutation negative normal and 12 replicates of mutation positive normal). Once again, there were no discrepant results yielding both intra-assay and inter-assay variability of zero.

**Table 2: Result of Reference Range Studies for Qualitative EGFR Assay**

| **Matrix** | **Variant** | **Median** | **Mean Current (nA)** | **SD** | **2 SD** | **3 SD** | **Reference Range (3SD)** |
|---|---|---|---|---|---|---|---|
| Plasma | T790M | -6.43771 | -5.96 | 16.18 | 32.37 | 48.55 | <43 nA |
| Plasma | L858R | -25.84727 | -22.79 | 14.07 | 28.14 | 42.21 | <20 nA |
| Plasma | Exon19 | -34.24165 | -28.05 | 15.90 | 31.79 | 47.69 | <20 nA |
| Saliva | T790M | 10.49715 | 8.82 | 11.58 | 23.16 | 34.75 | <44 nA |
| Saliva | L858R | -0.5126045 | -1.13 | 10.56 | 21.13 | 31.69 | <31 nA |
| Saliva | Exon19 | -3.927202 | -4.46 | 12.08 | 24.16 | 36.24 | <32 nA |

### Validation of Qualitative eLB Assay on Saliva

Figure 4 represents the same experiment using saliva samples obtained from consented healthy dentists at annual meetings of the American Dental Association. Plate design and validation design were identical to that of the plasma validation. The assay performance is similar to the plasma assay. Intra-assay and inter-assay variability were also zero with all replicates concordant for positive or negative results. As with plasma, the reference ranges vary slightly with each variant. Table 2 is a summary of the reference range determinations for the 6 assays, namely plasma and saliva for the 3 EGFR and Exon19del, p.L858R, and p.T790M. Although there are small variations in the reference range for each assay, positive clinical samples are well above these cut-off values. The internal positive controls are well above the 3SD cutoff for all variants.

As with any potential screening test, the reference range may need to be adjusted according to clinical performance. One advantage of eLB over other techniques is the ability to analyze both saliva and plasma from the same patient. Both tests can be performed simultaneously on each patient and those with positive results for both plasma and saliva are considered to be screen positive. For those individuals with one positive and one negative test, repeat samples are obtained and analyzed to resolve the discrepancy.

### Clinical Sensitivity and Specificity for eLB Qualitative Assay

The utility of any assay is determined by the sensitivity/specificity and positive and negative predictive values for the test. For the eLB platform, there is limited data on these parameters for the detection of *EGFR* variants in NSCLC patients. In 2 published studies involving late stage (III and IV) NSCLC patients, the data (Wei et al., 2014, Am J Respir Crit Care Med, 190:1117-1126; Pu et al., 2016, Thorac Cancer, 7:428-436) shows that qualitative eLB assays detected all 23 patients with p.L858R variants and all 15 patients with Exon 19del variants in their respective tumors. There were no false positives in 28 NSCLC patients whose tumors did not contain either of those variants. Therefore, qualitative eLB is used for treatment selection, with high sensitivity and specificity in these late stage NSLC patients.

A study of the performance of plasma qualitative eLB in early stage (I and II) NSCLC patients revealed a sensitivity of 92% for p.L858R and 77% for Exon 19del. The specificities were 95% for the two variants in a control group of patients with benign pulmonary nodules. Of note, the concordance was 100% between tissue variant analysis and eLB nodule biopsy. There were no discrepant results in these studies.

### eLB Quantitative Assays

The evaluation of the Quantitative eLB was done in 2 parts. Initially the limit of detection and linearity and linear range were established using genomic DNA controls. However, biological materials are not optimal for use as internal calibrators for assays. Therefore, following the establishment of limit of detection, linearity and linear range using genomic DNA, development of a clinical assay was performed using synthetic oligonucleotides as internal standards and calibrators.

### EGFR Exon 19del

The Exon 19del variant in *EGFR* is actually a family of closely related variants. Table 2 lists the 5 most prevalent variants in this family. Because the break points of these deletions are proximate to each other, it was possible to design a capture / detector probe set theoretically capable of detecting most, if not all of the Exon 19del family of variants (Table 3). In order to demonstrate that the eLB assay could detect the 4 most common Exon 19del variants were obtained DNA from 4 cell lines, each containing one of the common variants. The genomic DNA was treated and the placed into the eLB reaction at varying concentrations.

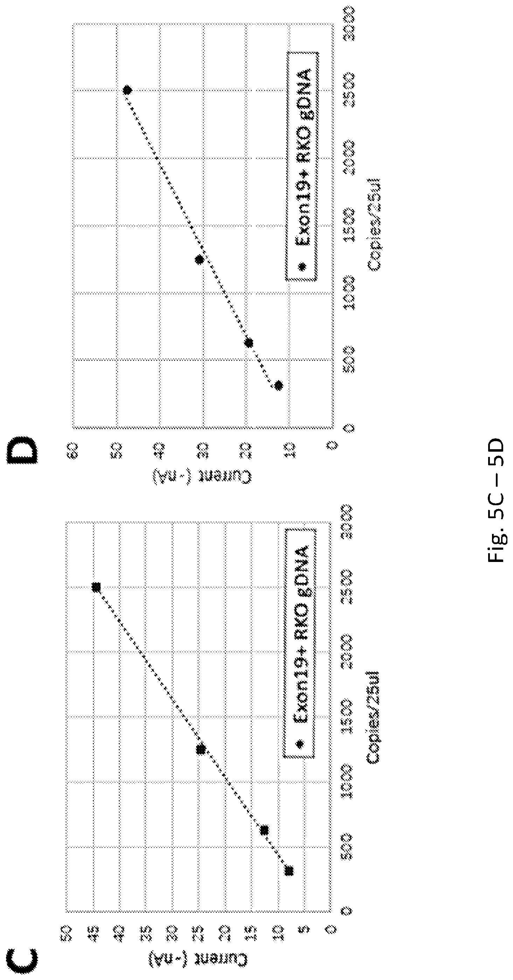

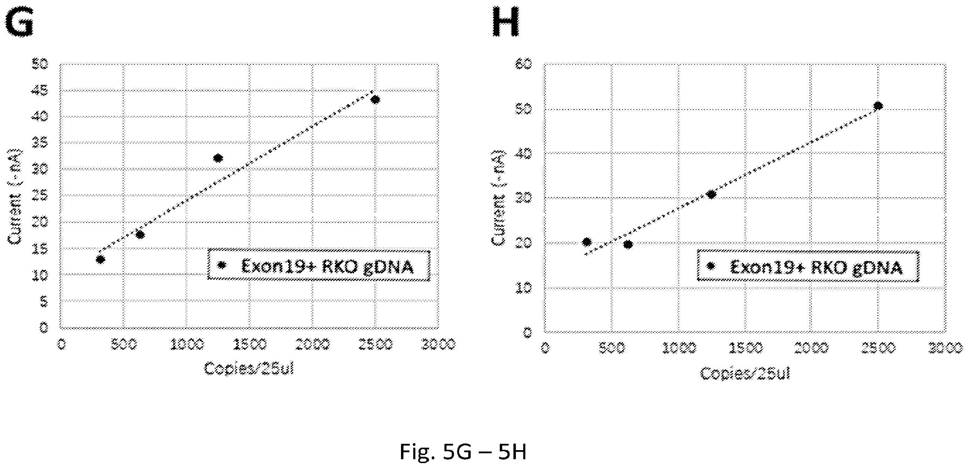

Figure 5 shows the results obtained using serial dilutions of genomic DNA containing the 4 most common EGFR 19del variants. As can be seen, the EGFR assay is capable of detecting all 4 of these variants with similar performance.

The assays are linear for all 4 variants with R² values of 0.99, 0.99, 0.95, and 0.94 respectively for variants c.2235_2249 del18, c2236_2250 del18, c2240 2257 del18, and c.2239 _2248 del11. The linear range extends from 20,000 copies / 25 µL to 500 copies / 25 µL. It is important to note, the sheared genomic DNA fragments are not the optimal template for the eLB reaction. Therefore, the lower limit of detection may be grossly underestimated. These data should, therefore, be considered as a minimal sensitivity for the eLB reaction.

**Table 3: List of common Exon 19del Variants**

| Amino Acid | Nucleotide | COSMIC # | Prevalence^{a} | Prevalence^{b} |
|---|---|---|---|---|
| p.E746_A750delE LREA (1) | c.2235_2249del18 | 6623 | 70% | 45% |
| p.E746_A750delE LREA (2 | c.2236_2250del18 | 6225 | | 29.6% |
| p.L747_P753>S | c.2240_2257del18 | 12370 | 5.9% | 8.4% |
| p.L747_A750>P | c.2239_2248TTA AGAGAAG>C | 12382 | 2.0% | 3.4% |
| p.L747_E749delL RE | c.2239_2247delT TAAGAGAA | 6218 | 0.3% | n/a |

| | | | | |
|---|---|---|---|---|
| ^{a} Cosmic Database: cancer.sanger.ac.uk/cosmic ^{b} Su et al., 2017, Oncotarget, 8:111246-111257 | | | | |

### Linearity and Limit of Detection of eLB for T790M

Figure 6 displays the data from various dilutions of genomic DNA containing the variant p.T790M. The assay is linear with and R² of 0.99 over a range of 300 to single digit copy number per 25 µL.

### Linearity at Limit of Detection of eLB for p.L858R

Figure 7 demonstrates the linearity and range for p.L858R. Linearity is excellent with an R² of 0.998 through a range of 300 copies to single digit copy number per 25 µL.

### Analytical Validation of eLB for 3 EGFR Variants

Once the linearity and LOD was determined for each variant separately, the performance of the eLB for the variants combined into a single assay was examined. One measure of the sensitivity of a liquid biopsy platform is the ability of the assay to detect variant sequences in a background of non-variant sequences. Reference materials are available for this purpose, containing artificial mixtures of wild type and variant sequences. Standards were purchased from Horizon Diagnostics and these samples were subjected to eLB analysis (see Figure 8). The data demonstrates the eLB results for the 3 *EGFR* variants and reveals that for T790M eLB is capable of detecting variants at a 0.1% level. The exon 19del and p.L858R are slightly less sensitive, but are able to clearly detect variant sequences at the 1% level. Standards were not available between 1% and 0.1% so it is not possible to determine how much lower than the 1% minor allele fraction EFRIM is capable of detecting for Exon 19del and p.L858R. These levels of detection are better than the in vitro diagnostic kit available from Roche for EGFR detection in liquid biopsies.

For the quantitative eLB three variant assays, synthetic oligonucleotide standards were used for calibration and internal standards. Titration in quadruplicate was performed using varying dilutions of oligonucleotides containing each of the 3 variants at concentrations of 0.02 pM to 1600 pM. Linearity was excellent with R² values of 0.99, 0.98 and 0.99 for p.T790M, p.L858R, and Exon 19del respectively.

### Validation Performance Results (Linearity, Inter and Intra-assay CV, Reference Range)

Quantitative validation of eLB was accomplished by performing a series of experiments with plasma and saliva matrices to evaluate assay linearity, assay CV, and reference range for p.L858R, Exon19 Deletion, and p.T790M. For linearity, two-fold dilutions ranging from 1000 pM to 24 femtomolar were run with synthetic oligonucleotide targets spiked into plasma or saliva matrices. Each dilution was performed 4 times. The results demonstrated a R² ≥0.99 for all p.L858R (R² = 0.99), Exon19 Deletion (R² = 0.99), and p.T790M (R² =0.99) when in the 0 to 10pM range, the 0 to 50pM range, and the 0 to 12.5 pM range, respectively in saliva. Similarly, an R² ≥0.99 was achieved for p.L858R (R² =0.996), Exon19 Deletion (R² =0.999), and p.T790m (R² = 0.996) in plasma in the range of 0 to 6.25 pM, 0 to 12.5pM, and 0 to 12.5pM range respectively. Limit of detection was calculated via a copy number to oligonucleotide target equivalence method, with the limit of detection of saliva being measured at 27.51 fM (3 copies), 144.86 fM (31 copies), 60.38 fM(9 copies) for the p.L858R, Exon19 Deletion, and p.T790M mutations, respectively and for plasma being 41.63 fM(119 copies), 28.26 fM (211 copies), and 27.72 fM (142 copies) respectively.

For intra-assay variation, for each mutation a plate of 88 replicates of a 3.25pM of oligonucleotide target spiked in biofluid with a 4-point standard curve (3.25 pM, 1.56pM, 0.78 pM, and 0 pM concentration). The intra-assay CV's were 8.72%, 18.6%, and 12.5% for p.L858R, Exon19 Deletion, and p.T790M respectively in plasma and 29.3%, 25.2%, and 23.3% in saliva respectively.

For inter-assay CV, four runs were made where each run assayed 24 replicates of each mutation at 3.25pM in biofluid and a 3-point standard curve. The Inter-assay CV's were 42.2%, 45.9%, and 30.7% for plasma and 40.6%, 35.9%, and 29.0% for saliva for p.L858R, Exon19 Deletion, and p.T790M respectively.

For each biofluid, healthy plasma and saliva from 40 healthy subjects were run across 4 or 5 plates for each of the three mutations with a 4-point standard curve in order to assess the variability of signal levels within a healthy population. Cutoff values were established based on the 3 standard deviations from the mean at 4.04 nA, 4.34 nA, 6.40 nA above normalized background current measurement for saliva and 7.10 nA, 9.18 nA, and 12.57 nA above normalized background current measurement for plasma for the p.L858R, Exon19 Deletion, and p.T790M respectively.

## Claims

1. A system for detecting a nucleic acid molecule of interest in a sample, comprising:
a) a multi-well plate comprising an array of sensors, wherein each well comprises an electrode chip including a working electrode, a counter electrode, and a reference electrode; wherein the working electrode of at least one unit is coated with a conducting polymer;
b) at least one probe set comprising a paired capture and detector probe,
wherein the capture probe comprises a polyA region and is embedded or functionalized in the conducting polymer and further wherein the detector probe is biotin labeled at the terminal 3'nucleotide;
c) a multi-well plate washer; and
d) a multi-channel electrochemical reader which controls an electrical field applied onto the array sensors and reports the amperometric current simultaneously.

2. The system of claim 1, wherein at least one of the capture and detector probes hybridize to a nucleic acid molecule comprising a marker of lung cancer.

3. The system of claim 1, wherein the polyA region comprises a sequence comprising at least 65 nucleotides, wherein at least 80% of the nucleotides are adenine, and wherein the polyA sequence is interrupted by non-adenine nucleotides.

4. The system of claim 2, wherein the marker of lung cancer is a variant of Epidermal growth factor receptor (EGFR) selected from the group consisting of Exon19 Deletion, T790M, and L858R;
wherein the paired capture and detection probes are selected from the group consisting of:
a) paired probes for detection of Exon19 Deletion, wherein the capture probe comprises a nucleotide sequence selected from the group consisting of SEQ ID NO:5 and SEQ ID NO: 13, and wherein the detector probe comprises SEQ ID NO:6;
b) paired probes for detection of L858R, wherein the capture probe comprises a nucleotide sequence selected from the group consisting of SEQ ID NO:1 and SEQ ID NO: 14, and wherein the detector probe comprises SEQ ID NO:2; and
c) paired probes for detection of T790M, wherein the capture probe comprises a nucleotide sequence selected from the group consisting of SEQ ID NO:9 and SEQ ID NO: 15, and wherein the detector probe comprises SEQ ID NO: 10.

5. A method of detecting lung cancer in a subject comprising:
a) providing at least one sample obtained from a subject;
b) mixing a first portion of the at least one sample with a solution comprising a biotin labeled detector probe;
c) adding the mixture to a single well of a multi-well plate for use in the system of claim 1, wherein each well of the multi-well plate comprises an electrode chip comprising a working electrode, a counter electrode, and a reference electrode; wherein the working electrode is coated with a conducting polymer embedded with a capture probe, wherein the capture probe comprises a polyA region;
d) applying a cyclic square-wave electric field to the electrode chip;
e) adding a first round of streptavidin bound horseradish peroxidase (HRP) to the well,
f) adding a biotin labeled anti-HRP antibody to the well,
g) adding a second round of streptavidin bound HRP to the well, and
h) measuring the current in the electrode chip, wherein a change in current is correlated to the presence of a marker associated with lung cancer in the sample.

6. The method of claim 5, further comprising at least one washing step, wherein the multi-well plate is washed using an automated plate washer.

7. The method of claim 5, wherein at least one reagent is maintained at 4°C prior to use.

8. The method of claim 5, wherein at least one of the capture and detector probes hybridize to a nucleic acid molecule comprising a marker of lung cancer.

9. The method of claim 8, wherein the nucleic acid molecule is a circulating tumor DNA (ctDNA) molecule.

10. The method of claim 5, wherein the polyA region comprises a sequence comprising at least 65 nucleotides, wherein at least 80% of the nucleotides are adenine, and wherein the polyA sequence is interrupted by non-adenine nucleotides.

11. The method of claim 5, wherein the marker of lung cancer is a variant of EGFR selected from the group consisting of Exon19 Deletion, T790M, and L858R;
wherein the paired capture and detection probes are selected from the group consisting of:
a) paired probes for detection of Exon19 Deletion, wherein the capture probe comprises a nucleotide sequence selected from the group consisting of SEQ ID NO:5 and SEQ ID NO:13, and wherein the detector probe comprises SEQ ID NO:6;
b) paired probes for detection of L858R, wherein the capture probe comprises a nucleotide sequence selected from the group consisting of SEQ ID NO:1 and SEQ ID NO:14, and wherein the detector probe comprises SEQ ID NO:2; and
c) paired probes for detection of T790M, wherein the capture probe comprises a nucleotide sequence selected from the group consisting of SEQ ID NO:9 and SEQ ID NO:15, and wherein the detector probe comprises SEQ ID NO:10.

12. The method of claim 5, wherein at least one sample is selected from the group consisting of a saliva sample, a blood sample, a plasma sample and a serum sample.

13. The method of claim 5, wherein a saliva sample from a subject is added to a first well of the multi-well plate and a plasma sample from the same subject is added to a second well of the multi-well plate.

14. The method of claim 13, wherein a subject is diagnosed as having or being at risk lung cancer when a maker of lung cancer is detected in both the saliva sample from the subject and the plasma sample from the same subject.

## Patentansprüche

1. System zum Nachweisen eines Nukleinsäuremoleküls von Interesse in einer Probe, umfassend:
a) eine Multiwell-Platte, die ein Array von Sensoren umfasst, wobei jedes Well einen Elektrodenchip umfasst, der eine Arbeitselektrode, eine Gegenelektrode und eine Referenzelektrode enthält; wobei die Arbeitselektrode mindestens einer Einheit mit einem leitfähigen Polymer beschichtet ist;
b) mindestens einen Sondensatz, der eine gepaarte Einfang- und Detektorsonde umfasst, wobei die Einfangsonde eine PolyA-Region umfasst und im leitfähigen Polymer eingebettet oder funktionalisiert ist und weiter wobei die Detektorsonde am 3'-terminalen Nukleotid mit Biotin markiert ist;
c) eine Multiwell-Platten-Waschvorrichtung; und
d) ein mehrkanaliges elektrochemisches Lesegerät, das ein an die Arraysensoren angelegtes elektrisches Feld steuert und den amperometrischen Strom gleichzeitig anzeigt.

2. System nach Anspruch 1, wobei mindestens eine der Einfang- und Detektorsonden an ein Nukleinsäuremolekül hybridisiert, das einen Marker für Lungenkrebs umfasst.

3. System nach Anspruch 1, wobei die PolyA-Region eine mindestens 65 Nukleotide umfassende Sequenz umfasst, wobei es sich bei mindestens 80 % der Nukleotide um Adenin handelt und wobei die PolyA-Sequenz durch Nicht-Adenin-Nukleotide unterbrochen ist.

4. System nach Anspruch 2, wobei es sich bei dem Marker für Lungenkrebs um eine Variante des Epidermaler-Wachstumsfaktor-Rezeptors (Epidermal Growth Factor Receptor, EGFR) handelt, die aus der Gruppe bestehend aus Exon19-Deletion, T790M und L858R ausgewählt ist;
wobei die gepaarten Einfang- und Nachweissonden ausgewählt sind aus der Gruppe bestehend aus:
a) gepaarten Sonden zum Nachweis von Exon19-Deletion, wobei die Einfangsonde eine Nukleotidsequenz umfasst, die aus der Gruppe bestehend aus SEQ ID NO:5 und SEQ ID NO:13 ausgewählt ist, und wobei die Detektorsonde SEQ ID NO:6 umfasst;
b) gepaarten Sonden zum Nachweis von L858R, wobei die Einfangsonde eine Nukleotidsequenz umfasst, die aus der Gruppe bestehend aus SEQ ID NO:1 und SEQ ID NO:14 ausgewählt ist, und wobei die Detektorsonde SEQ ID NO:2 umfasst; und
c) gepaarten Sonden zum Nachweis von T790M, wobei die Einfangsonde eine Nukleotidsequenz umfasst, die aus der Gruppe bestehend aus SEQ ID NO:9 und SEQ ID NO:15 ausgewählt ist, und wobei die Detektorsonde SEQ ID NO:10 umfasst.

5. Verfahren zum Nachweis von Lungenkrebs bei einem Individuum, umfassend:
a) Bereitstellen mindestens einer von einem Individuum erhaltenen Probe;
b) Mischen eines ersten Teils der mindestens einen Probe mit einer Lösung, die eine mit Biotin markierte Detektorsonde umfasst;
c) Füllen des Gemischs in ein einzelnes Well einer Multiwell-Platte zur Verwendung in dem System nach Anspruch 1, wobei jedes Well der Multiwell-Platte einen Elektrodenchip umfasst, der eine Arbeitselektrode, eine Gegenelektrode und eine Referenzelektrode umfasst; wobei die Arbeitselektrode mit einem leitfähigen Polymer mit einer darin eingebetteten Einfangsonde beschichtet ist, wobei die Einfangsonde eine PolyA-Region umfasst;
d) Anlegen eines zyklischen elektrischen Rechteckwellenfelds an den Elektrodenchip;
e) Zugeben einer ersten Runde an Streptavidin gebundener Meerrettich-Peroxidase (HRP) in das Well,
f) Zugeben eines mit Biotin markierten Anti-HRP-Antikörpers in das Well,
g) Zugeben einer zweiten Runde an Streptavidin gebundener HRP in das Well und
h) Messen des Stroms im Elektrodenchip, wobei eine Stromänderung mit dem Vorliegen eines mit Lungenkrebs assoziierten Markers in der Probe korreliert ist.

6. Verfahren nach Anspruch 5, ferner umfassend mindestens einen Waschschritt, wobei die Multiwell-Platte unter Verwendung eines Plattenwaschautomaten gewaschen wird.

7. Verfahren nach Anspruch 5, wobei mindestens ein Reagens vor der Verwendung bei 4 °C gehalten wird.

8. Verfahren nach Anspruch 5, wobei mindestens eine der Einfang- und Detektorsonden an ein Nukleinsäuremolekül hybridisiert, das einen Marker für Lungenkrebs umfasst.

9. Verfahren nach Anspruch 8, wobei es sich bei dem Nukleinsäuremolekül um ein ctDNA(circulating tumor DNA)-Molekül handelt.

10. Verfahren nach Anspruch 5, wobei die PolyA-Region eine mindestens 65 Nukleotide umfassende Sequenz umfasst, wobei es sich bei mindestens 80 % der Nukleotide um Adenin handelt und wobei die PolyA-Sequenz durch Nicht-Adenin-Nukleotide unterbrochen ist.

11. Verfahren nach Anspruch 5, wobei es sich bei dem Marker für Lungenkrebs um eine EGFR-Variante handelt, die aus der Gruppe bestehend aus Exon19-Deletion, T790M und L858R ausgewählt ist;
wobei die gepaarten Einfang- und Nachweissonden ausgewählt sind aus der Gruppe bestehend aus:
a) gepaarten Sonden zum Nachweis von Exon19-Deletion, wobei die Einfangsonde eine Nukleotidsequenz umfasst, die aus der Gruppe bestehend aus SEQ ID NO:5 und SEQ ID NO:13 ausgewählt ist, und wobei die Detektorsonde SEQ ID NO:6 umfasst;
b) gepaarten Sonden zum Nachweis von L858R, wobei die Einfangsonde eine Nukleotidsequenz umfasst, die aus der Gruppe bestehend aus SEQ ID NO:1 und SEQ ID NO:14 ausgewählt ist, und wobei die Detektorsonde SEQ ID NO:2 umfasst; und
c) gepaarten Sonden zum Nachweis von T790M, wobei die Einfangsonde eine Nukleotidsequenz umfasst, die aus der Gruppe bestehend aus SEQ ID NO:9 und SEQ ID NO:15 ausgewählt ist, und wobei die Detektorsonde SEQ ID NO:10 umfasst.

12. Verfahren nach Anspruch 5, wobei mindestens eine Probe aus der Gruppe bestehend aus einer Speichelprobe, einer Blutprobe, einer Plasmaprobe und einer Serumprobe ausgewählt ist.

13. Verfahren nach Anspruch 5, wobei eine Speichelprobe von einem Individuum in ein erstes Well der Multiwell-Platte und eine Plasmaprobe von demselben Individuum in ein zweites Well der Multiwell-Platte gegeben wird.

14. Verfahren nach Anspruch 13, wobei bei einem Individuum Lungenkrebs oder ein Lungenkrebsrisiko diagnostiziert wird, wenn sowohl in der Speichelprobe von dem Individuum als auch in der Plasmaprobe von demselben Individuum ein Marker für Lungenkrebs nachgewiesen wird.

## Revendications

1. Système de détection d'une molécule d'acide nucléique d'intérêt dans un échantillon, comprenant :
a) une plaque multi-puits comprenant un réseau de capteurs, dans laquelle chaque puits comprend une puce d'électrode incluant une électrode de travail, une contre-électrode et une électrode de référence ; dans laquelle l'électrode de travail d'au moins une unité est revêtue d'un polymère conducteur ;
b) au moins un ensemble de sondes comprenant une sonde de capture et de détecteur appariée, dans lequel la sonde de capture comprend une région polyA et est incorporée ou fonctionnalisée dans le polymère conducteur et en outre dans lequel la sonde de détecteur est marquée par de la biotine au niveau du nucléotide 3' terminal ;
c) un laveur de plaques multi-puits ; et
d) un lecteur électrochimique multicanal qui commande un champ électrique appliqué sur les capteurs de réseau et rapporte simultanément le courant ampérométrique.

2. Système selon la revendication 1, dans lequel au moins l'une des sondes de capture et de détection s'hybride avec une molécule d'acide nucléique comprenant un marqueur de cancer du poumon.

3. Système selon la revendication 1, dans lequel la région polyA comprend une séquence comprenant au moins 65 nucléotides, dans lequel au moins 80 % des nucléotides sont l'adénine, et dans lequel la séquence polyA est interrompue par des nucléotides non adénine.

4. Système selon la revendication 2, dans lequel le marqueur de cancer du poumon est un variant du récepteur de facteur de croissance épidermique (EGFR) choisi dans le groupe constitué par délétion d'Exon19, T790M, et L858R ;
dans lequel les sondes de capture et de détection appariées sont choisies dans le groupe constitué par :
a) des sondes appariées pour la détection de la délétion d'Exon19, dans lesquelles la sonde de capture comprend une séquence nucléotidique choisie dans le groupe constitué par SEQ ID NO:5 et SEQ ID NO:13, et dans lesquelles la sonde de détecteur comprend SEQ ID NO:6 ;
b) des sondes appariées pour la détection de L858R, dans lesquelles la sonde de capture comprend une séquence nucléotidique choisie dans le groupe constitué par SEQ ID NO:1 et SEQ ID NO:14, et dans lesquelles la sonde de détecteur comprend SEQ ID NO:2 ; et
c) des sondes appariées pour la détection de T790M, dans lesquelles la sonde de capture comprend une séquence nucléotidique choisie dans le groupe constitué par SEQ ID NO:9 et SEQ ID NO:15, et dans lesquelles la sonde de détecteur comprend SEQ ID NO:10.

5. Procédé de détection de cancer du poumon chez un sujet comprenant :
a) fourniture d'au moins un échantillon obtenu à partir d'un sujet ;
b) mélange d'une première partie de l'au moins un échantillon avec une solution comprenant une sonde de détecteur marquée par de la biotine ;
c) ajout du mélange à un seul puits d'une plaque multi-puits pour une utilisation dans le système selon la revendication 1, dans lequel chaque puits de la plaque multi-puits comprend une puce d'électrode comprenant une électrode de travail, une contre-électrode, et une électrode de référence ; dans lequel l'électrode de travail est revêtue d'un polymère conducteur intégré avec une sonde de capture, la sonde de capture comprenant une région polyA ;
d) application d'un champ électrique à ondes carrées cycliques sur la puce d'électrode ;
e) ajout d'un premier cycle de peroxydase de raifort (HRP) liée à la streptavidine au puits,
f) ajout d'un anticorps anti-HRP marqué par de la biotine au puits,
g) ajout d'un second cycle de HRP liée à la streptavidine au puits, et
h) mesure du courant dans la puce d'électrode, un changement de courant étant corrélé à la présence d'un marqueur associé à un cancer du poumon dans l'échantillon.

6. Procédé selon la revendication 5, comprenant en outre au moins une étape de lavage, dans lequel la plaque multi-puits est lavée en utilisant un laveur de plaques automatisé.

7. Procédé selon la revendication 5, dans lequel au moins un réactif est maintenu à 4 °C avant utilisation.

8. Procédé selon la revendication 5, dans lequel au moins l'une des sondes de capture et de détecteur s'hybride avec une molécule d'acide nucléique comprenant un marqueur de cancer du poumon.

9. Procédé selon la revendication 8, dans lequel la molécule d'acide nucléique est une molécule d'ADN tumoral circulant (ADNtc).

10. Procédé selon la revendication 5, dans lequel la région polyA comprend une séquence comprenant au moins 65 nucléotides, dans lequel au moins 80 % des nucléotides sont l'adénine, et dans lequel la séquence polyA est interrompue par des nucléotides non adénine.

11. Procédé selon la revendication 5, dans lequel le marqueur de cancer du poumon est un variant d'EGFR choisi dans le groupe constitué par délétion d'Exon19, T790M, et L858R ;
dans lequel les sondes de capture et de détection appariées sont choisies dans le groupe constitué par :
a) des sondes appariées pour la détection de la délétion d'Exon19, dans lesquelles la sonde de capture comprend une séquence nucléotidique choisie dans le groupe constitué par SEQ ID NO:5 et SEQ ID NO:13, et dans lesquelles la sonde de détecteur comprend SEQ ID NO:6 ;
b) des sondes appariées pour la détection de L858R, dans lesquelles la sonde de capture comprend une séquence nucléotidique choisie dans le groupe constitué par SEQ ID NO:1 et SEQ ID NO:14, et dans lesquelles la sonde de détecteur comprend SEQ ID NO:2 ; et
c) des sondes appariées pour la détection de T790M, dans lesquelles la sonde de capture comprend une séquence nucléotidique choisie dans le groupe constitué par SEQ ID NO:9 et SEQ ID NO:15, et dans lesquelles la sonde de détecteur comprend SEQ ID NO:10.

12. Procédé selon la revendication 5, dans lequel au moins un échantillon est choisi dans le groupe constitué par un échantillon de salive, un échantillon de sang, un échantillon de plasma et un échantillon de sérum.

13. Procédé selon la revendication 5, dans lequel un échantillon de salive provenant d'un sujet est ajouté à un premier puits de la plaque multi-puits et un échantillon de plasma provenant du même sujet est ajouté à un second puits de la plaque multi-puits.

14. Procédé selon la revendication 13, dans lequel un sujet est diagnostiqué comme ayant ou étant à risque de cancer du poumon lorsqu'un marqueur de cancer du poumon est détecté à la fois dans l'échantillon de salive du sujet et l'échantillon de plasma du même sujet.
